# EUROPEAN PATENT APPLICATION

(11) **EP 4 640 076 A1**
(43) Date of publication of application: **29.10.2025**
(21) Application number: 24171616.6
(22) Date of filing: 22.04.2024
(51) Int. Cl.: A24B 15/16, A24B 15/30, A61K 9/107

(54) **ORAL PRODUCT WITH FLAVOR EMULSION**

(71) Applicant: Nicoventures Trading Limited, London WC2R 3LA (GB)
(72) Inventor: Poole, Thomas H., Southampton (GB); Suresh, Kaviya, Southampton (GB); Pinto, M. Isabel, Southampton (GB)
(74) Representative: Newcombe, Christopher David

(57) **Abstract**

The disclosure provides an emulsion including a solvent, a lipid component, an emulsifying agent having a hydrophilic-lipophilic balance (HLB) value in a range from about 3 to about 17, a lipophilic flavoring agent, and an active ingredient having a logP value in a range from about -0.5 to about 3.5. Further provided are compositions including such an emulsion and one or more fillers, pouched oral products including a pouch and the composition enclosed therein. Also provided are methods of preparing such emulsions, compositions, and pouched oral products.

## Description

### FIELD OF THE DISCLOSURE

The present disclosure relates to an oral product. In particular, the present disclosure relates to products intended for human consumption. The products are configured for oral use and deliver substances such as flavors and/or active ingredients during use.

### BACKGROUND

There are many categories of products intended for oral use and enjoyment. For example, oral tobacco products containing nicotine, which is known to have both stimulant and anxiolytic properties, have been available for many years. Conventional formats for so-called "smokeless" tobacco products include moist snuff, snus, and chewing tobacco, which are typically formed almost entirely of particulate, granular, or shredded tobacco, and which are either portioned by the user or presented to the user in individual portions, such as in single-use pouches or sachets. See for example, the types of smokeless tobacco formulations, ingredients, and processing methodologies set forth in US Pat. Nos. 6,668,839 to Williams; 6,834,654 to Williams; 6,953,040 to Atchley et al.; 7,032,601 to Atchley et al.; and 7,694,686 to Atchley et al.; 7,810,507 to Dube et al.; 7,819,124 to Strickland et al.; 7,861,728 to Holton, Jr. et al.; 7,901,512 to Quinter et al.; 8,627,828 to Strickland et al.; 11,246,334 to Atchley, each of which is incorporated herein by reference.

In addition, traditional tobacco materials and non-tobacco materials have been combined with other ingredients to form product formats distinct from traditional smokeless products, with example formats including lozenges, pastilles, gels, and the like. See, for example, the types of products described in US Patent App. Pub. Nos. 2008/0196730 to Engstrom et al.; 2008/0305216 to Crawford et al.; 2009/0293889 to Kumar et al.; 2010/0291245 to Gao et al; 2011/0139164 to Mua et al.; 2012/0037175 to Cantrell et al.; 2012/0055494 to Hunt et al.; 2012/0138073 to Cantrell et al.; 2012/0138074 to Cantrell et al.; 2013/0074855 to Holton, Jr.; 2013/0074856 to Holton, Jr.; 2013/0152953 to Mua et al.; 2013/0274296 to Jackson et al.; 2015/0068545 to Moldoveanu et al.; 2015/0101627 to Marshall et al.; and 2015/0230515 to Lampe et al., each of which is incorporated herein by reference.

There is continuing interest in the development of new types of oral products that deliver advantageous sensorial or biological activity. Such products typically contain flavorants and/or active ingredients such as nicotine, caffeine, botanicals, or cannabidiol. The format of such products can vary and include pouched products containing a powdered or granular composition, lozenges, pastilles, liquids, gels, emulsions, meltable compositions, and the like. See, for example, the types of products described in US Patent App. Pub. Nos. 2022/0160675 to Gerardi et al.; 2022/0071984 to Poole et al.; 2021/0378948 to Gerardi et al.; 2021/0330590 to Hutchens et al.; 2021/0186081 to Gerardi et al.; 2021/0177754 to Keller et al; 2021/0177043 to Gerardi et al.; 2021/0177038 to Gerardi et al.; 2021/0169867 to Holton, Jr. et al.; 2021/0169792 to Holton, Jr. et al.; 2021/0169132 to Holton, Jr. et al.; 2021/0169121 to St. Charles, and 2021/0169122 to St. Charles, each of which is incorporated herein by reference.

### BRIEF SUMMARY

The disclosure generally provides an emulsion, and a composition configured for oral use comprising such an emulsion, one or more fillers, and optionally, one or more sweeteners; one or more salts, and one or more buffering agents. Further provided is a method of preparing such emulsions and compositions. Accordingly, in one aspect is provided an emulsion comprising a solvent, a lipid component, an emulsifying agent, and an active ingredient. In another aspect is provided a composition comprising the emulsion and one or more fillers, and optionally, one or more sweeteners, one or more salts, and one or more buffering agents. Further provided is a pouched oral product comprising a pouch and the composition enclosed therein. In another aspect is provided are methods of preparing emulsions, compositions, and pouched products as described herein. In some embodiments the method of preparing the emulsion generally comprises combining an emulsifying agent, a lipid component, a flavoring agent, and an active ingredient to form a self-emulsifying delivery system (SEDS) component; providing a buffer solution comprising a buffering agent and a solvent; combining the SEDS component with the buffer solution to form an emulsion. In some embodiments, the method further comprises combining the emulsion with one or more of a fillers, sweeteners, and salts to form the composition. The method of forming the emulsion is advantageous in providing emulsions which are generally stable and clear or translucent. Surprisingly, it has been found that the order of combining the emulsion components is important to provide a stable emulsion having a desirable particle size range (i.e., such that the emulsion is optically clear or slightly translucent). Specifically, it has been found that combining the lipid component, flavoring agent, emulsifier, and active ingredient together first to form a SEDS component, followed by combining this SEDS component with water and buffer, provides an emulsion with desirable properties. In contrast, adding a combination of the lipid component, flavoring agent, and emulsifier to a water phase comprising buffer and active ingredient does not provide an emulsion with the desired properties. This is particularly surprising in that the relatively hydrophilic active ingredients utilized in the present disclosure would not have been expected to destabilize an emulsion. Further, the relatively hydrophilic active ingredients actually stabilize the emulsion when added as part of the SEDS component and reduces the quantity of emulsifying agent necessary to achieve a stable emulsion having a desirable particle size range.

The disclosure includes, without limitations, the following embodiments.

Embodiment 1: A method of preparing an emulsion configured for oral use, the method comprising: combining an emulsifying agent, a lipid component, a flavoring agent, and an active ingredient having a logP value in a range from about -0.5 to about 3.5, to form a self-emulsifying delivery system (SEDS) component; providing a buffer solution comprising a buffering agent and a solvent; and combining the SEDS component with the buffer solution to form the emulsion.

Embodiment 2: The method of embodiment 1, wherein the emulsifying agent has a hydrophilic-lipophilic balance (HLB) value in a range from about 3 to about 17, such as from about 3 to about 5, from about 8 to about 12, or from about 13 to about 17.

Embodiment 3: The method of embodiment 1 or 2, wherein the emulsifying agent comprises a polyethylene glycol monoester of a fatty acid; optionally, wherein the polyethylene glycol monoester of a fatty acid is a polyoxyethylene sorbitan monooleate, and preferably, wherein the polyoxyethylene sorbitan monooleate is polysorbate 20, polysorbate 40, polysorbate 60, polysorbate 80, or a combination thereof.

Embodiment 4: The method of embodiment 1 or 2, wherein the emulsifying agent comprises lecithin, gum acacia, modified gum acacia, a modified starch, or a combination thereof.

Embodiment 5: The method of any one of embodiments 1-4, wherein the lipid component comprises medium chain triglycerides.

Embodiment 6: The method of any one of embodiments 1-5, wherein the lipid component comprises acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, okra seed oil, olive oil, orange oil, palm oil, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, soybean oil, sunflower oil, taramira oil, tea seed oil, thistle oil, tigernut oil, tobacco seed oil, tomato seed oil, walnut oil, watermelon seed oil, wheat germ oil, or a combination thereof; optionally, wherein the lipid component comprises castor oil, corn oil, coconut oil, cod liver oil, evening primrose oil, cottonseed oil, palm oil, rice bran oil, sesame oil, rapeseed oil, canola oil, linseed oil, olive oil, peanut oil, soybean oil, safflower oil, flaxseed oil, sunflower oil, olive oil, or a combination thereof.

Embodiment 7: The method of any one of embodiments 1-6, wherein the flavoring agent is lipophilic.

Embodiment 8: The method of any one of embodiments 1-7, wherein the flavoring agent comprises one or more volatile components, one or more components readily oxidizable upon exposure to air, or both; optionally, wherein the flavoring agent comprises heptanal.

Embodiment 9: The method of any one of embodiments 1-8, wherein the active ingredient is nicotine, a kavalactone, melatonin, or a combination thereof; optionally, wherein the active ingredient is nicotine.

Embodiment 10: The method of any one of embodiments 1-9, wherein the solvent comprises water, propylene glycol, glycerol, 1,2-propanediol, or a combination thereof.

Embodiment 11: The method of any one of embodiments 1-10, wherein the buffering agent comprises a bicarbonate or carbonate alkali metal salt; preferably, wherein the buffering agent is sodium bicarbonate.

Embodiment 12: The method of any one of embodiments 1-11, wherein combining the emulsifying agent, the lipid component, the flavoring agent, and the active ingredient; and combining the SEDS component with the buffer solution, each comprise stirring the respective components for a period of time at a speed in a range from about 100 to about 1000 rpm.

Embodiment 13: The method of any one of embodiments 1-12, wherein the emulsion comprises:
about 60% by weight or higher of the solvent, based on the total weight of the emulsion;
about 40% by weight or lower of the SEDS component, based on the total weight of the emulsion; and
wherein a ratio by weight of the emulsifying agent to the lipid component is in a range from about 3:1 to about 5:1.

Embodiment 14: The method of any one of embodiments 1-13, further comprising combining the emulsion with a powder component comprising one or more of a filler, a sweetener, and a salt to form a composition configured for oral use.

Embodiment 15: The method of embodiment 14, wherein the filler comprises a cellulose material; optionally, wherein the filler comprises microcrystalline cellulose.

Embodiment 16: The method of embodiment 14 or 15, wherein the composition comprises water in an amount from about 15 to about 25% by weight, based on the total weight of the composition.

Embodiment 17: The method of any one of embodiments 14-16, further comprising:
enclosing the composition in a pouch to form a pouched product; and
adding a quantity of water and/or humectant to the pouched product, wherein the quantity of water and/or humectant added is sufficient to provide an oven volatile content of the pouched product of about 5% to about 60%, such as about 40% to about 60% by weight, based on the total weight of the pouched product.

Embodiment 18: An oral product comprising a composition, the composition comprising:
an emulsion comprising a solvent, a lipid component, an emulsifying agent having a hydrophilic-lipophilic balance (HLB) value in a range from about 3 to about 17, a lipophilic flavoring agent, and an active ingredient having a logP value in a range from about -0.5 to about 3.5;
one or more fillers; and
optionally, one or more sweeteners; one or more salts, and one or more buffering agents.

Embodiment 19: The pouched oral product of embodiment 18, comprising:
from about 30 to about 60% of the filler;
from about 40 to about 60% by weight of water;
from about 1 to about 5% by weight of emulsifying agent;
from about 0.25 to about 10% by weight of the lipid component;
from about 0.1 to about 10% by weight of active ingredient; and
from about 0.01 to about 2% by weight of the lipophilic flavoring agent, wherein each of the quantities by weight are based on the total weight of the pouched oral product.

Embodiment 20: The oral product of embodiment 18 or 19, wherein the composition is substantially free of alginates and sugar alcohols; preferably, wherein the composition is completely free of alginates and xylitol.

Embodiment 21: The oral product of any one of embodiments 18-20, wherein the lipid component comprises medium chain triglycerides.

Embodiment 22: The oral product of any one of embodiments 18-21, wherein the buffering agent is a carbonate or bicarbonate salt of an alkali metal; optionally, wherein the buffering agent is sodium bicarbonate.

Embodiment 23: The oral product of any one of embodiments 18-22, wherein the one or more fillers is a cellulose material; optionally, wherein the filler comprises microcrystalline cellulose.

Embodiment 24: The oral product of any one of embodiments 18-23, wherein the active ingredient comprises nicotine, a kavalactone, melatonin, resveratrol, or a combination thereof; optionally, wherein the active ingredient is nicotine.

Embodiment 25: The oral product of any one of embodiments 18-24, wherein a ratio by weight of the emulsifying agent to the lipid component is in a range from about 3:1 to about 5:1.

Embodiment 26: The oral product of any one of embodiments 18-25, wherein a combination of the flavoring agent and the lipid comprises the flavoring agent in an amount of about 20% by weight or more, based on a total combined weight of the lipid and the flavoring agent; optionally, wherein the combination of the flavoring agent and the lipid comprises the flavoring agent in an amount of about 25% or more or about 30% or more by weight, based on a total combined weight of the lipid and the flavoring agent.

Embodiment 27: The oral product of any one of embodiments 18-26, wherein the lipid component comprises acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, okra seed oil, olive oil, orange oil, palm oil, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, soybean oil, sunflower oil, taramira oil, tea seed oil, thistle oil, tigernut oil, tobacco seed oil, tomato seed oil, walnut oil, watermelon seed oil, wheat germ oil, or a combination thereof; optionally, wherein the lipid component comprises castor oil, corn oil, coconut oil, cod liver oil, evening primrose oil, cottonseed oil, palm oil, rice bran oil, sesame oil, rapeseed oil, canola oil, linseed oil, olive oil, peanut oil, soybean oil, safflower oil, flaxseed oil, sunflower oil, olive oil, or a combination thereof.

Embodiment 28: The oral product of any one of embodiments 18-27, wherein the flavoring agent is lipophilic.

Embodiment 29: The oral product of any one of embodiments 18-28, wherein the flavoring agent comprises one or more volatile components, one or more components readily oxidizable upon exposure to air, or both; optionally, wherein the flavoring agent comprises heptanal.

Embodiment 30: The oral product of any one of embodiments 18-29, wherein the emulsifying agent has a hydrophilic-lipophilic balance (HLB) value in a range from about 3 to about 17, such as from about 3 to about 5, from about 8 to about 12, or from about 13 to about 17.

Embodiment 31: The oral product of any one of embodiments 18-30, wherein the emulsifying agent comprises a polyethylene glycol monoester of a fatty acid; optionally, wherein the polyethylene glycol monoester of a fatty acid is a polyoxyethylene sorbitan monooleate, and preferably, wherein the polyoxyethylene sorbitan monooleate is polysorbate 20, polysorbate 40, polysorbate 60, polysorbate 80, or a combination thereof.

Embodiment 32: The oral product of any one of embodiments 18-31, wherein the emulsifying agent comprises lecithin, gum acacia, modified gum acacia, a modified starch, or a combination thereof.

Embodiment 33: An oral product comprising a composition comprising:
an emulsion comprising a solvent, a lipid component, an emulsifying agent having a hydrophilic-lipophilic balance (HLB) value in a range from about 3 to about 17, a lipophilic flavoring agent, and an active ingredient, wherein a ratio by weight of the emulsifying agent to the lipid component is in a range from about 3:1 to about 5:1;
one or more fillers; and
optionally, one or more sweeteners; one or more salts, and one or more buffering agents.

Embodiment 34: The oral product of any one of embodiments 18-33, wherein the composition is enclosed in a water-permeable pouch to form a pouched product.

These and other features, aspects, and advantages of the disclosure will be apparent from a reading of the following detailed description together with the accompanying drawings, which are briefly described below. The disclosure includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosure, in any of its various aspects and embodiments, should be viewed as intended to be combinable unless the context clearly dictates otherwise.

### BRIEF DESCRIPTION OF THE DRAWINGS

Having thus described aspects of the disclosure in the foregoing general terms, reference will now be made to the accompanying drawings, which are not necessarily drawn to scale. The drawings are exemplary only and should not be construed as limiting the disclosure.
**FIG. 1** is a perspective view of a pouched product embodiment according to an example embodiment of the present disclosure including a pouch or fleece at least partially filled with a composition according to a non-limiting embodiment of the disclosure.
**FIG. 2** is a graphical depiction of viscosity versus shear rate for examples according to non-limiting embodiments of the disclosure.
**FIG. 3** is a graphical depiction of volatile flavor component concentration over time for a pouched example according to non-limiting embodiments of the disclosure.

### DETAILED DESCRIPTION

The present disclosure will now be described more fully hereinafter with reference to example embodiments thereof. These example embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements.

As used in this specification and the claims, the singular forms "a," "an," and "the" include plural referents unless the context clearly dictates otherwise.

The term "about" used throughout this specification is used to describe and account for small fluctuations. For example, the term "about" can refer to less than or equal to ±10%, such as less than or equal to ±5%, less than or equal to ±2%, less than or equal to ±1%, less than or equal to ±0.5%, less than or equal to ±0.2%, less than or equal to ±0.1% or less than or equal to ±0.05%. All numeric values herein are modified by the term "about," whether or not explicitly indicated. A value modified by the term "about" of course includes the specific value. For instance, "about 5.0" must include 5.0.

Reference to "dry weight percent" or "dry weight basis" refers to weight on the basis of dry ingredients (i.e., all ingredients except water). Reference to "wet weight" refers to the weight of the composition including water. Unless otherwise indicated, reference to "weight percent" of a composition reflects the total wet weight of the composition (i.e., including water).

By "substantially free" is meant that none of a particular component has been intentionally added, beyond trace amounts that may be present e.g., as an impurity in another component. For example, some embodiments can be characterized as having less than 0.001% by weight of the specified component, or less than 0.0001%, or even 0% by weight of the specified component, based on the total weight of the emulsion or composition. In some embodiments the composition may be completely free of a specified component (e.g., having 0% by weight of the specified component, based on the total weight of the composition).

The present disclosure generally provides an emulsion, a composition comprising such emulsion, and a pouched oral product comprising a pouch and a composition enclosed therein. The composition comprises an emulsion; one or more fillers; and optionally, one or more sweeteners; one or more salts; and one or more buffering agents. Further provided is a method of preparing such emulsions, compositions and oral products. The example individual components of the emulsion, the composition, the overall product, and the method of making the composition are each described herein below.

### Emulsion

In one aspect is provided an emulsion comprising a solvent, a lipid component, an emulsifying agent having a hydrophilic-lipophilic balance (HLB) value in a range from about 3 to about 17, a lipophilic flavoring agent, and an active ingredient (e.g., an active ingredient having a logP value in a range from about -0.5 to about 3.5). Such an emulsion serves to protect the flavoring agent against oxidation and evaporation and may enhance the dissolution and/or absorption of the active ingredient. In such emulsions, the oil (comprising the flavoring agent and the active agent) is the dispersed phase, and a solvent such as water is the continuous phase. The oil phase is present as droplets with varying size. In some embodiments, the emulsion is a nanoemulsion. By "nanoemulsion" is meant a colloidal particulate system with particulates in the sub-micron size range, for example particulates having an average size of less than about 1,000 nm, such as from about 10 to about 1,000 nm, or from about 100 to about 300 nm. The particulates (also referred to herein as droplets) are generally spherical, and the surfaces of such droplets are generally hydrophilic, while the interiors are generally lipophilic. Each of the emulsion components (e.g., solvent, lipid component, flavoring agent, active ingredient, emulsifying agent) are described further herein below.

### Lipid component

The emulsion comprises a lipid component. The lipid component is generally an oil. Any suitable oil may be used, including petroleum-based (e.g., mineral oil) and natural or naturally derived oils (e.g., from plant materials or animal sources). Generally, the oil is a food-grade oil. Such oils include, but are not limited to, vegetable oils (e.g., acai oil, almond oil, amaranth oil, apricot oil, apple seed oil, argan oil, avocado oil, babassu oil, beech nut oil, ben oil, bitter gourd oil, black seed oil, black currant seed oil, borage seed oil, borneo tallow nut oil, bottle gourd oil, brazil nut oil, buffalo gourd oil, butternut squash seed oil, cape chestnut oil, canola oil, carob cashew oil, cocoa butter, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, nutmeg butter, okra seed oil, olive oil, orange oil, palm oil, palm stearin, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, shea butter, soybean oil, sunflower oil, taramira oil, tea seed oil, thistle oil, tigernut oil, tobacco seed oil, tomato seed oil, walnut oil, watermelon seed oil, wheat germ oil, and combinations thereof), animal oils (e.g., cattle fat, buffalo fat, sheep fat, goat fat, pig fat, lard, camel fat, tallow, liquid margarine, fish oil, fish liver oil, whale oil, seal oil, and combinations thereof), and mineral oils.

In some embodiments, the lipid component is a natural, food-grade oil. In some embodiments, the oil is a natural or naturally derived oil. In some embodiments, the oil comprises a long chain fatty acid or a medium chain fatty acid, such as the long chain fatty acid or medium chain fatty acid or one or more of a glycerol mono-, di-, or triester of either thereof (i.e., a monoacylglycerol, diacylglycerol, triacylglycerol, or a combination thereof, wherein the acyl group is a medium or long chain fatty acid).

As used herein, "medium chain fatty acid" refers to a carboxylic (CO₂H) acid having an aliphatic carbon chain of from about 6 to about 11 carbon atoms. The aliphatic carbon chain may be straight or branched. The aliphatic carbon chain may be saturated (i.e., having all sp3 carbon atoms), or may be unsaturated (i.e., having at least one site of unsaturation). As used herein, the term "unsaturated" refers to the presence of a carbon-carbon, sp² double bond in one or more positions within the aliphatic carbon chain. Unsaturated alkyl groups may be mono- or polyunsaturated. Representative medium chain fatty acids include, but are not limited to, caproic acid, caprylic acid, decanoic acid, and undecanoic acid.

As used herein, "long chain fatty acid" refers to a carboxylic (CO₂H) acid having an aliphatic carbon chain of from about 11 to about 21 carbon atoms. The aliphatic carbon chain may be straight or branched. The aliphatic carbon chain may be saturated (i.e., having all sp³ carbon atoms), or may be unsaturated (i.e., having at least one site of unsaturation). As used herein, the term "unsaturated" refers to the presence of a carbon-carbon, sp² double bond in one or more positions within the aliphatic carbon chain. Unsaturated alkyl groups may be mono- or polyunsaturated. Representative long chain fatty acids include, but are not limited to undecylic acid, undecanoic acid, lauric acid, tridecanoic acid, myristic acid, pentadecanoic acid, palmitic acid, margaric acid, stearic acid, nonadecanoic acid, arachidic acid, heneicosanoic acid, α-linolenic acid, stearidonic acid, eicosapentaenoic acid, cervonic acid, linoleic acid, linolelaidic acid, γ-linolenic acid, dihomo-γ-linolenic acid, and arachidonic acid.

In some embodiments, the oil comprises a triacylglycerol, wherein the acyl group is a medium chain fatty acid as described herein. In some embodiments, the oil comprises a triacylglycerol, wherein the acyl group is a medium chain fatty acid as described herein. Such medium chain fatty acid triacylglycerols may also be referred to herein as "medium chain triglycerides" or "MCTs." In some embodiments, the oil is enriched in MCT's, meaning the oil has a greater concentration of MCTs relative to other triglycerides, such as short or long chain triglycerides. In some embodiments, the oil is MCT oil, such as a commercially available MCT oil derived from coconut or palm kernel oil by fractionation.

In some embodiments, the oil comprises a triacylglycerol, wherein the acyl group is a long chain fatty acid as described herein. Such long chain fatty acid triacylglycerols may also be referred to herein as "long chain triglycerides" or "LCTs." In some embodiments, the oil is enriched in LCT's, meaning the oil has a greater concentration of LCTs relative to other triglycerides, such as short or medium chain triglycerides. In some embodiments, the LCTs comprise a relatively large percentage of unsaturated long chain fatty acids (e.g., oleic and/or linoleic acids).

The amount of lipid component present within the emulsion and the overall composition can vary. In some embodiments, the composition, or a pouched product comprising the composition, comprises the lipid component in an amount by weight from about 0.25 to about 10% by weight, based on the total weight of the pouched oral product.

### Emulsifying Agent

The emulsion comprises at least one emulsifying agent. As used herein, the term "emulsifying agent" refers to a substance which aids in the formation and stabilization of emulsions by promoting dispersion of hydrophobic and hydrophilic (e.g., oil and water) components. In general, emulsifying agents are amphiphilic molecules chosen from, for example, nonionic and ionic amphiphilic molecules. The expression "amphiphilic molecule" means any molecule of bipolar structure comprising at least one hydrophobic portion and at least one hydrophilic portion and having the property of reducing the surface tension of water and of reducing the interface tension between water and an oily phase. Emulsifying agents/amphiphilic molecules as provided herein are also referred to as, for example, surfactants and emulsifiers.

Suitable emulsifying agents include small molecule surfactants, phospholipids, proteins, polysaccharides, and mixtures thereof. Examples of suitable emulsifying agents include, but are not limited to, polyethylene glycol esters of fatty acids, propylene glycol esters of fatty acids, polysorbates, polyglycerol esters of fatty acids, polyglycerol polyricinoleate, sorbitan esters of fatty acid, sucrose esters of fatty acids, lecithins, enzyme treated lecithins, glycerin fatty acids esters, acetic acid esters of monoglycerides, lactic acid esters of monoglycerides, citric acid esters of monoglycerides, succinic acid esters of monoglycerides, diacetyl tartaric acid esters of monoglycerides, calcium stearoyl di lactate, chitin and chitosan derivatives, natural and modified starches, natural and modified hydrocolloids, natural and modified polysaccharides, natural and modified celluloses, natural and modified proteins, synthetic amphiphilic polymers, glycol distearate, sorbitan trioleate, sorbitan tristearate, sorbitan triisostearate, glyceryl isostearate, propylene glycol isostearate, glycol stearate, sorbitan sesquioleate, glyceryl stearate, lecithin, sorbitan oleate, sorbitan monostearate, sorbitan stearate, sorbitan isostearate, steareth-2, oleth-2, PEG-7 hydrogenated castor oil, laureth-2, sorbitan palmitate, laureth-3, glyceryl laurate, ceteth-2, PEG-30 dipolyhdroxystearate, glyceryl stearate SE, sorbitan stearate (and) sucrose cocoate, PEG-4 dilaurate, methyl glucose sesquistearate, PEG-8 dioleate, sorbitan laurate, PEG-40 sorbitan peroleate, laureth-4, PEG-7 glyceryl cocoate, PEG-20 almond glycerides, PEG-25 hydrogenated castor oil, stearamide MEA, glyceryl stearate, PEG-100 stearate, polysorbate 81, polysorbate 85, polysorbate 65, PEG-7 glyceryl cocoate, PEG-8 stearate, PEG-8 caprate, PEG-35 almond glycerides, PEG-6 laurate, laureth-7, steareth-10, isotrideceth-8, PEG-35 castor oil, isotrideceth-9, PEG-40 castor oil, ceteareth-12, laureth-9, PEG-40 hydrogenated castor oil, PEG-20 glyceryl isostearate, PEG-20 stearate, PEG-40 sorbitan perisostearate, PEG-7 olivate, cetearyl glucoside, PEG-8 oleate, polyglyceryl-3 methylglucose distearate, oleth-10, oleth-10/polyoxyl 10 oleyl ether NF, ceteth-10, PEG-8 laurate, cocamide MEA, polysorbate 60, polysorbate 80, isosteareth-20, PEG-60 almond glycerides, PEG-20 methyl glucose sesquistearate, ceteareth-20, oleth-20, steareth-20, steareth-21, ceteth-20, isoceth-20, polysorbate 20, polysorbate 40, ceteareth-25, ceteareth-30, PEG-30 stearate, laureth-23, PEG-75 lanolin, polysorbate 20, PEG-40 stearate, PEG-100 stearate, steareth-100, PEG-80 sorbitan laurate, polyoxyethylene stearate (e.g., polyoxyethylene (40) stearate), polyoxyethylene ether, and mixtures thereof.

In some embodiments, the emulsifying agent comprises one or more of polyethylene glycol esters of fatty acids, propylene glycol esters of fatty acids, polysorbates, polyglycerol esters of fatty acids, polyglycerol polyricinoleate, sorbitan esters of fatty acid, sucrose esters of fatty acids, lecithins, enzyme treated lecithins, glycerin fatty acids esters, acetic acid esters of monoglycerides, lactic acid esters of monoglycerides, citric acid esters of monoglycerides, succinic acid esters of monoglycerides, diacetyl tartaric acid esters of monoglycerides, calcium stearoyl di lactate, chitin and chitosan derivatives, nature and modified starches, nature and modified hydrocolloids, nature and modified polysaccharides, nature and modified celluloses, nature and modified proteins, synthetic amphiphilic polymers, or mixtures thereof.

In some embodiments, the emulsifying agent comprises: polyoxyethylene-sorbitan-fatty acid ester; e.g., mono- and tri-lauryl, palmityl, stearyl and oleyl esters; e.g., products of the type known as polysorbates and commercially available under the trade name Tween^{®}; polyoxyethylene fatty acid esters, e.g., polyoxyethylene stearic acid esters of the type known and commercially available under the trade name Myrj^{®}; polyoxyethylene castor oil derivatives, e.g., products of the type known and commercially available as Cremophors^{®}; PEG glyceryl fatty acid esters such as PEG-8 glyceryl caprylate/caprate (commercially known as Labrasol^{®}), PEG-4 glyceryl caprylate/caprate (Labrafac Hydro WL 1219), PEG-32 glyceryl laurate (Gelucire 44/14), PEG-6 glyceryl mono oleate (Labrafil^{®} M 1944 CS), PEG-6 glyceryl linoleate (Labrafil^{®} M 2125 CS); propylene glycol mono- and di-fatty acid esters, such as propylene glycol laurate, propylene glycol caprylate/caprate; sorbitan fatty acid esters, such as the type known and commercially available under the name Span^{®} (e.g., Span 85); polyoxyethylene-polyoxypropylene co-polymers, e.g., products of the type known and commercially available as Pluronic^{®} or Poloxamer^{®}; glycerol triacetate; monoglycerides and acetylated monoglycerides, e.g., glycerol monodicocoate (Imwitor^{®} 928), glycerol monocaprylate (Imwitor^{®} 308), mono-and di-acetylated monoglycerides; or combinations thereof
In some embodiments, the emulsifying agent has an HLB value in a range from about 3 to about 17. As will be understood by one skilled in the art, HLB is the hydrophilic-lipophilic balance of an emulsifying agent and is a measure of the degree to which it is hydrophilic or lipophilic. The HLB value may be determined in accordance with the industry standard textbook, namely "The HLB SYSTEM, a time-saving guide to emulsifier selection" ICI Americas Inc., Published 1976 and Revised, March, 1980. The HLB values of the emulsifiers described herein were determined in accordance with this standard method.

Examples of suitable emulsifying agents having an HLB value in the range from about 3 to about 17 include, but are not limited to, mono and diglycerides of fatty acid including glyceryl stearate and glyceryl oleate; fatty acid esters of C12-C22 fatty alcohols including fatty acid esters of cetyl alcohol and fatty acid esters of stearoyl alcohol, mixtures of fatty acid esters of cetyl alcohol and fatty acid esters of stearoyl alcohol, mixtures of fatty acid esters of cetyl alcohol and fatty acid esters of stearoyl alcohol wherein the fatty acids are derived from olive oil (such as cetearyl olivate), fatty acid esters of sorbitol including sorbitan oleate, fatty acid esters of sorbitol wherein the fatty acids are derived from olive oil (such as sorbitan olivate or cetearyl olivate), lecithin, certain modified starches, certain gums including modified gums, and mixtures of any thereof.

The selection of emulsifying agent may depend at least in part on the method utilized in forming the emulsion (e.g., low or high energy as described herein below).

In some embodiments, the emulsifying agent comprises lecithin, gum acacia, modified gum acacia, a modified starch, or a combination thereof.

In some embodiments, the emulsifying agent comprises a polyethylene glycol monoester of a fatty acid. In some embodiments, the polyethylene glycol monoester of a fatty acid is a polyoxyethylene sorbitan monooleate, such as polysorbate 20, polysorbate 40, polysorbate 60, polysorbate 80, or a combination thereof. In some embodiments, the emulsifying agent is polysorbate 80.

In some embodiments, the emulsion is prepared by a high-energy method as described herein below. Accordingly, in some embodiments, the emulsifying agent comprises one or more of Gum Acacia, Modified gum Arabic, Modified Starch, Lecithin, Polyoxyl esters of fatty acids, Sucrose Esters of fatty acids, Citric Acid Esters of Mono- and Di-glycerides, Lactylic esters of fatty acids, Phosphorylated mono and di-glycerides, glycol distearate, sorbitan trioleate, sorbitan tristearate, sorbitan triisostearate, glyceryl isostearate, propylene glycol isostearate, glycol stearate, sorbitan sesquioleate, glyceryl stearate, lecithin, sorbitan oleate, sorbitan monostearate, sorbitan stearate, sorbitan isostearate, steareth-2, oleth-2, PEG-7 hydrogenated castor oil, laureth-2, sorbitan palmitate, laureth-3, glyceryl laurate, ceteth-2, PEG-30 dipoly hdroxystearate, glyceryl stearate SE, sorbitan stearate (and) sucrose cocoate, PEG-4 dilaurate, methyl glucose sesquistearate, PEG-8 dioleate, sorbitan laurate, PEG-40 sorbitan peroleate, laureth-4, PEG-7 glyceryl cocoate, PEG-20 almond glycerides, PEG-25 hydrogenated castor oil, stearamide MEA, glyceryl stearate (and) PEG-100 stearate, polysorbate 81, polysorbate 85, polysorbate 65, PEG-7 glyceryl cocoate, PEG-8 stearate, PEG-8 caprate, PEG-35 almond glycerides, PEG-6 laurate, laureth-7, steareth-10, isotrideceth-8, PEG-35 castor oil, isotrideceth-9, PEG-40 castor oil, ceteareth-12, laureth-9, PEG-40 hydrogenated castor oil, PEG-20 glyceryl isostearate, PEG-20 stearate, PEG-40 sorbitan perisostearate, PEG-7 olivate, cetearyl glucoside, PEG-8 oleate, polyglyceryl-3 methylglucose distearate, oleth-10, oleth-10/polyoxyl 10 oleyl ether NF, ceteth-10, PEG-8 laurate, cocamide MEA, polysorbate 60, polysorbate 80, isosteareth-20, PEG-60 almond glycerides, PEG-20 methyl glucose sesquistearate, ceteareth-20, oleth-20, steareth-20, steareth-21, ceteth-20, isoceth- 20, polysorbate 20, polysorbate 40, ceteareth-25, ceteareth-30, PEG-30 stearate, laureth-23, PEG-75 lanolin, polysorbate 20, PEG-40 stearate, PEG-100 stearate, steareth-100, PEG-80 sorbitan laurate, polyoxyethylene stearate (e.g. polyoxyethylene (40) stearate), polyoxyethylene ether, polyethylene glycol esters of fatty acids, propylene glycol esters of fatty acids, polysorbates, poly glycerol esters of fatty acids, poly glycerol polyricinoleate, sorbitan esters of fatty acid, sucrose esters of fatty acids, lecithins, enzyme treated lecithins, glycerin fatty acids esters, acetic acid esters of monoglycerides, lactic acid esters of monoglycerides, citric acid esters of monoglycerides, succinic acid esters of monoglycerides, diacetyl tartaric acid esters of monoglycerides, calcium stearoyl di lactate, chitin and chitosan derivatives, nature and modified starches, nature and modified hydrocolloids, natural and modified polysaccharides, natural and modified celluloses, natural and modified proteins, synthetic amphiphilic polymers, and mixtures thereof.

The amount of the emulsifying agent(s) present in the emulsion may vary. The amount of emulsifying agent present may depend at least in part on the method utilized in forming the emulsion (e.g., low or high energy as described herein below). In some embodiments, the quantity of emulsifying agent present in the emulsion may be expressed as a ratio to the lipid component. In some embodiments, the ratio by weight of the emulsifying agent to the lipid component is in a range from about 3:1 to about 5:1, such as about 3:1, about 4:1, or about 5:1 by weight.

In some embodiments, a composition, or a pouched product comprising the composition, each as described herein below, comprises from about 0.1 to about 5% by weight of emulsifying agent, such as from about 0.1, about 0.5, or about 1%, to about 2%, about 3%, about 4%, or about 5% by weight, based on the total weight of the composition, or pouched product.

### Flavoring Agent

The emulsion comprises one or more flavoring agents. In some embodiments, one or more flavoring agents are present in the oil-in-water emulsion, and the composition further comprises one or more flavoring agents, which may be the same or may be different from the flavoring agent present in the emulsion. As used herein, the term "flavoring agent" or "flavorant" refers to any flavorful or aromatic substance capable of altering the sensory characteristics associated with the composition. Examples of sensory characteristics that can be modified by the flavoring agent include taste, mouthfeel, moistness, coolness/heat, and/or fragrance/aroma. Flavoring agents may be natural or synthetic, and the character of the flavors imparted thereby may be described, without limitation, as fresh, sweet, herbal, confectionary, floral, fruity, or spicy. Generally, the flavoring agent is lipophilic, meaning it is relatively more soluble in fats and oils, and relatively less soluble in water.

Specific types of flavors provided by flavoring agents include, but are not limited to, apple, anise, cardamom, cascarilla cherry, chocolate/cocoa, cinnamon, clove, coffee, cream, eucalyptus, ginger, honey, jasmine, lavender, lemon, licorice, lime, menthol, mint, nutmeg, orange, peach, peppermint, pineapple, sage, sandalwood, spearmint, strawberry, vanilla, wintergreen, and any combinations thereof. See also, Leffingwell et al., Tobacco Flavoring for Smoking Products, R. J. Reynolds Tobacco Company (1972), which is incorporated herein by reference. Flavorings may include components that are considered moistening, cooling or smoothening agents, such as eucalyptus. These flavors may be provided neat (i.e., alone) or in a composite, and may be employed as concentrates or flavor packages (e.g., spearmint and menthol, orange and cinnamon, lime, tropical, and the like). Representative types of components also are set forth in US Pat. No. 5,387,416 to White et al.; US Pat. App. Pub. No. 2005/0244521 to Strickland et al.; and PCT Application Pub. No. WO 05/041699 to Quinter et al., each of which is incorporated herein by reference. In some embodiments, a portion of the flavoring agent may be provided in a spray-dried form or may be in a liquid form.

The flavoring agent generally comprises at least one volatile flavor component. As used herein, "volatile" refers to a chemical substance that forms a vapor readily at ambient temperatures (i.e., a chemical substance that has a high vapor pressure at a given temperature relative to a nonvolatile substance). Generally, volatile flavor components are those having a vapor pressure greater than about 0.02 mm of mercury at 25°C. Typically, a volatile flavor component has a molecular weight below about 400 and will often include at least one carbon-carbon double bond, carbon-oxygen double bond, carbon-oxygen single bond, or combinations thereof. In some embodiments, the at least one volatile flavor component comprises one or more alcohols, alkanes, alkenes, aldehydes, aromatic hydrocarbons, ketones, esters, terpenes, terpenoids, or combinations thereof. Non-limiting examples of aldehydes include vanillin, ethyl vanillin, p-anisaldehyde, heptanal, hexanal, furfural, isovaleraldehyde, cuminaldehyde, benzaldehyde, and citronellal. Non-limiting examples of ketones include 1-hydroxy-2-propanone and 2-hydroxy-3-methyl-2-cyclopentenone-1-one. Non-limiting examples of terpenes include sabinene, limonene, gamma-terpinene, beta-farnesene, nerolidol, thujone, myrcene, geraniol, nerol, citronellol, linalool, and eucalyptol. Non-limiting examples of esters include isoamyl acetate, ethyl hexanoate, ethyl butyrate, and octyl acetate.

The amount of flavorant utilized can vary, but is typically up to about 10% by weight, and some embodiments are characterized by a flavoring agent content of at least about 0.1% by weight, such as about 0.1% to about 10% by weight, or about 1 to about 10% by weight, based on the total weight of the emulsion.

In some embodiments, the emulsion comprises a combination of the flavoring agent and the lipid where the flavoring agent is present in an amount of about 20% by weight or more, based on a total combined weight of the lipid and the flavoring agent. In some embodiments, the combination of the flavoring agent and the lipid comprises the flavorant in an amount of about 25% or more or about 30% or more by weight, based on a total combined weight of the lipid and the flavoring agent.

In some embodiments, a composition, or a pouched product comprising the composition, each as described herein below, comprises from about 0.01 to about 2% by weight of the lipophilic flavoring agent.

### Active Ingredient

The emulsion comprises an active ingredient. In some embodiments, an active ingredient is present in the emulsion, and the composition further comprises one or more active ingredients, which may be the same or may be different from the active ingredient present in the emulsion.

As used herein, the term "active ingredient" refers to one or more substances belonging to any of the following categories: API (active pharmaceutical substances), food additives, natural medicaments, and naturally occurring substances that can have an effect on humans. Example active ingredients include any ingredient known to impact one or more biological functions within the body, such as ingredients that furnish pharmacological activity or other direct effect in the diagnosis, cure, mitigation, treatment, or prevention of disease, or which affect the structure or any function of the body of humans (e.g., provide a stimulating action on the central nervous system, have an energizing effect, an antipyretic or analgesic action, or an otherwise useful effect on the body).

Non-limiting examples of active ingredients include those falling in the categories of stimulants, nicotine components, and/or pharmaceutical, nutraceutical, and medicinal ingredients). The particular choice of active ingredients will vary depending upon the desired flavor, texture, and desired characteristics of the particular oral product.

In some embodiments, the active ingredient has low to moderate lipophilicity. Lipophilicity is conveniently measured in terms of logP, the partition coefficient of a molecule between a lipophilic phase and an aqueous phase, usually octanol and water, respectively. LogP values may be measured experimentally according to protocols well known to one of skill in the art. Alternatively, logP values may be calculated using commercially available software.

In some embodiments, the active ingredient has a logP value in a range from about -0.5 to about 3.5, such as about 1, about 1.5, about 2, about 2.5, or about 3. In some embodiments, the active ingredient has a logP from about -0.5 to about 2.5, or about -0.5 to about 2, or about -0.5 to about 1.5. In some embodiments, the active ingredient has a logP of about -0.5, about 0, about 0.5, about 1, about 1.5, about 2, about 2.5, about 3, about 3.1, about 3.2, about 3.3, about 3.4, or about 3.5, or in a range between any two of these values.

In some embodiments, the active ingredient is nicotine, a kavalactone, melatonin, resveratrol, or a combination thereof.

In some embodiments, the active ingredient comprises nicotine. Typically, nicotine is present in the free base form. The source of the nicotine may vary and may be natural or synthetic. Nicotine may be tobacco-derived (e.g., a tobacco extract) or non-tobacco derived (e.g., synthetic or otherwise obtained). In some embodiments, the nicotine is naturally occurring and obtained as an extract from a *Nicotiana* species (e.g., tobacco). The nicotine can have the enantiomeric form S(-)-nicotine, *R*(+)-nicotine, or a mixture of S(-)-nicotine and *R*(+)-nicotine. In some embodiments, the nicotine is in the form of S(-)-nicotine (e.g., in a form that is virtually all S(-)-nicotine) or a racemic mixture composed primarily or predominantly of S(-)-nicotine (e.g., a mixture composed of about 95 weight parts S(-)-nicotine and about 5 weight parts *R*(+)-nicotine). Typically, the nicotine is employed in virtually pure form or in an essentially pure form. In some embodiments, nicotine that is employed has a purity of greater than about 95 percent, such as greater than about 98 percent, or greater than about 99 percent, on a weight basis. Nicotine has a calculated logP of about 1.2.

In some embodiments, the active ingredient comprises melatonin (N-[2-(5-methoxy-1H-indol-3-yl)ethyl]acetamide). Melatonin is a naturally occurring indoleamine hormone with a role in synchronizing circadian rhythms, including sleep-wake timing, blood pressure regulation, and control of seasonal rhythmicity. In addition to its role as a hormone, melatonin is used as a dietary supplement and medication in the treatment of sleep disorders such as insomnia and circadian rhythm sleep disorders such as delayed sleep phase disorder, jet lag disorder, and shift work disorder. Melatonin has a calculated logP of about 1.2.

In some embodiments, the active ingredient comprises a kavalactone, such as kavain. Kavalactones such as kavain have been isolated from the kava plant (*Piper methysticum*), and exhibit anxiolytic, sedative, and/or analgesic effects. Kavain (4-methoxy-6-[(E)-2-phenylethenyl]-5,6-dihydro-2H-pyran-2-one) is the major kavalactone obtained from the roots of the kava plant. Kavain has a calculated logP of about 2.1.

In some embodiments, the active ingredient comprises resveratrol (3,5,4'-trihydroxy-trans-stilbene). Resveratrol is a lipophilic (logP 3.1) naturally occurring phenolic stilbene found in certain fruits such as grapes, blueberries, and raspberries. Over the past 20 or so years, a variety of studies have suggested resveratrol may have antioxidant activity and may be beneficial in addressing cardiovascular disease, diabetes, enhancing cognitive function, and reducing effects of aging.

The particular percentage of active ingredient present in the emulsion will vary depending upon the desired characteristics of the specific emulsion and the format of the composition comprising the emulsion. Typically, an active ingredient or combination thereof is present in a total concentration of at least about 0.001% by weight of the emulsion or the oral product (e.g., a pouched oral product), such as in a range from about 0.001% to about 20%.

In some embodiments, the emulsion or a composition or product comprising the emulsion is substantially free of cannabinoids. In some embodiments, the emulsion or a composition or product comprising the emulsion comprises less than 0.001% by weight of cannabinoids, such as less than 0.0001%, or even 0% by weight of cannabinoids, based on the total weight of the emulsion, composition, or product. In some embodiments the emulsion or a composition or product comprising the emulsion is completely free of cannabinoids.

Nicotine, when present, is typically at a concentration of at least about 0.001% by weight of the overall composition (i.e., emulsion plus any other components such as fillers), such as in a range from about 0.001% to about 10%. In some embodiments, nicotine is present in a concentration from about 0.1% w/w to about 10% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, or about 10% by weight, calculated as the free base and based on the total weight of the composition.

In some embodiments, the active ingredient comprises a basic amine functionality (i.e., the active ingredient comprises a basic amine). By "basic amine" is meant a molecule including at least one basic amine functional group. Examples of basic amines include, but are not limited to, alkaloids such as nicotine. In some embodiments, the active ingredient comprising a basic amine functionality is nicotine. By "basic amine functional group" is meant a group containing a nitrogen atom having a lone pair of electrons. The basic amine functional group is attached to or incorporated within the molecule through one or more covalent bonds to the said nitrogen atom. The basic amine may be a primary, secondary, or tertiary amine, meaning the nitrogen bears one, two, or three covalent bonds to carbon atoms. By virtue of the lone pair of electrons on the nitrogen atom, such amines are termed "basic", meaning the lone electron pair is available for hydrogen bonding. The basicity (i.e., the electron density on the nitrogen atom and consequently the availability and strength of hydrogen bonding to the nitrogen atom) of the basic amine may be influenced by the nature of neighboring atoms, the steric bulk of the molecule, and the like.

Generally, an active ingredient comprising a basic amine functionality is released from the composition and absorbed through the oral mucosa, thereby entering the blood stream, where it is circulated systemically. In some embodiments, the active ingredient having a basic amine functionality is nicotine.

In some embodiments, at least a portion the active ingredient having the basic amine functionality (e.g., nicotine) is associated with an organic acid or an alkali metal salt thereof. Depending on multiple variables (concentration, pH, nature of the organic acid, and the like), the active ingredient having the basic amine functionality present in the composition can exist in multiple forms, including ion paired, in solution (i.e., fully solvated), as the free base, as a cation, as a salt, or any combination thereof. In some embodiments, at least a portion of the active ingredient present in the composition and having the basic amine functionality is in an ion paired form with an organic acid or a conjugate base thereof as further described herein below. Ion pairing is further described in, for example, International Patent Application Publication No. WO2021/050741 to Poole et al., and US Application Publication Nos. 2021/0068447 to Keller et al., 2023/0138306A1 to Zawadzki et al., and 2022/0346434 to Von Cosmos et al., each of which is incorporated herein by reference.

Ion pairing describes the partial association of oppositely charged ions in relatively concentrated solutions to form distinct chemical species called ion pairs. The strength of the association (i.e., the ion pairing) depends on the electrostatic force of attraction between the positive and negative ions (i.e., a protonated basic amine and the conjugate base of an organic acid). By "conjugate base" is meant the base resulting from deprotonation of the corresponding acid (e.g., benzoate is the conjugate base of benzoic acid).

On average, a certain population of these ion pairs exists at any given time, although the formation and dissociation of ion pairs is continuous. In the composition as disclosed herein, and/or upon oral use of said composition (e.g., upon contact with saliva), the active ingredient having the basic amine functionality and the conjugate base of the organic acid exist at least partially in the form of an ion pair. Without wishing to be bound by theory, it is believed that such ion pairing may minimize chemical degradation of the active ingredient having the basic amine functionality and/or enhance the oral availability of the active ingredient having the basic amine functionality. At alkaline pH values (e.g., such as from about 7.5 to about 9), certain active ingredient having the basic amine functionality, for example nicotine, are largely present in the free base form, which has relatively low water solubility, and low stability with respect to evaporation and oxidative decomposition, but high mucosal availability. Conversely, at acidic pH values (such as from about 6.5 to about 4), certain active ingredient having the basic amine functionality, for example nicotine, are largely present in a protonated form, which has relatively high water solubility, and higher stability with respect to evaporation and oxidative decomposition, but low mucosal availability. In some embodiments, the properties of stability, solubility, and availability of nicotine can be enhanced through ion pairing or salt formation of nicotine with appropriate organic acids and/or their conjugate bases.

One of skill in the art will recognize that the extent of ion pairing in the disclosed emulsion, both before and during use by the consumer, may vary based on, for example, pH, the nature of the acid, the concentration of active ingredient having the basic amine functionality (e.g., nicotine), the concentration of the acid or conjugate base of the acid present in the emulsion, the water content of the emulsion, the ionic strength of the emulsion, and the like. One of skill in the art will also recognize that ion pairing is an equilibrium process influenced by the foregoing variables. Accordingly, quantification of the extent of ion pairing is difficult or impossible by calculation or direct observation. However, the presence of ion pairing may be demonstrated through surrogate measures, such as partitioning of the active ingredient having the basic amine functionality (e.g., nicotine) between octanol and water, or by performing membrane permeation studies of aqueous solutions of the active ingredient having the basic amine functionality (e.g., nicotine) plus acids and/or their conjugate bases. An octanol-water partitioning favoring distribution of an ion pair into octanol is predictive of good absorption of the active ingredient having the basic amine functionality (e.g., nicotine) through the oral mucosa.

In embodiments where ion pairing is desired, the emulsion comprises an organic acid, an alkali metal salt of an organic acid, or both. In such embodiments, at least a portion of the active ingredient having the basic amine functionality (e.g., nicotine) is associated with at least a portion of the organic acid, the alkali metal salt thereof, or a combination thereof in the form an ion pair.

As used herein, the term "organic acid" refers to an organic (i.e., carbon-based) compound that is characterized by acidic properties. Typically, organic acids are relatively weak acids (i.e., they do not dissociate completely in the presence of water), such as carboxylic acids (-CO₂H) or sulfonic acids (-SO₂OH). As used herein, reference to organic acid means an organic acid that is intentionally added. In this regard, an organic acid may be intentionally added as a specific emulsion ingredient as opposed to merely being inherently present as a component of another ingredient (e.g., the small amount of organic acid which may inherently be present in an emulsion ingredient).

### Organic acid

In embodiments where ion pairing is desired, the emulsion further comprises an organic acid as defined herein above, and/or an alkali metal salt thereof. Suitable organic acids for ion pairing will typically have a range of lipophilicities (i.e., a polarity giving an appropriate balance of water and organic solubility). Typically, lipophilicities of suitable organic acids, as indicated by logP, will vary between about 1 and about 12 (more soluble in octanol than in water). In some embodiments, the organic acid has a logP value from about 1 to about 12, e.g., from about 1.0. about 1.5, about 2.0, about 2.5, about 3.0, about 3.5, about 4.0, about 4.5, about 5.0, about 5.5, about 6.0, about 6.5, about 7.0, about 7.5, or about 8.0, to about 8.5, about 9.0, about 9.5, about 10.0, about 10.5, about 11.0, about 11.5, or about 12.0.

Without wishing to be bound by theory, it is believed that moderately lipophilic organic acids (e.g., logP of from about 1.4 to about 4.5) produce ion pairs which are of a polarity providing good octanol-water partitioning of the ion pair, and hence partitioning of the active ingredient having the basic amine functionality (e.g., nicotine), into octanol versus water. As discussed above, such partitioning into octanol is predictive of favorable oral availability.

In specific embodiments, the organic acid has a logP value from about 3.0 to about 8.0, about 10.0, or even 12.0. In some embodiments, the presence of certain solvents or solubilizing agents (e.g., inclusion in the emulsion of glycerin or propylene glycol) may be beneficial in solubilizing organic acids and the corresponding salts or ion pairs thereof for highly lipophilic organic acids (e.g., higher than about 4.5).

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms (C₁-C₂₀). In some embodiments, the organic acid is an alkyl, cycloalkyl, heterocycloalkyl, aryl, or heteroaryl carboxylic or sulfonic acid.

As used herein, "alkyl" refers to any straight chain or branched chain hydrocarbon. The alkyl group may be saturated (i.e., having all *sp³* carbon atoms), or may be unsaturated (i.e., having at least one site of unsaturation). As used herein, the term "unsaturated" refers to the presence of a carbon-carbon, *sp²* double bond in one or more positions within the alkyl group. Unsaturated alkyl groups may be mono- or polyunsaturated. Representative straight chain alkyl groups include, but are not limited to, methyl, ethyl, n-propyl, n-butyl, n-pentyl, and n-hexyl. Branched chain alkyl groups include, but are not limited to, isopropyl, sec-butyl, isobutyl, tert-butyl, isopentyl, and 2-methylbutyl. Representative unsaturated alkyl groups include, but are not limited to, ethylene or vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like. An alkyl group can be unsubstituted or substituted.

"Cycloalkyl" as used herein refers to a carbocyclic group, which may be mono- or bicyclic. Cycloalkyl groups include rings having 3 to 7 carbon atoms as a monocycle or 7 to 12 carbon atoms as a bicycle. Examples of monocyclic cycloalkyl groups include cyclopropyl, cyclobutyl, cyclopentyl, cyclohexyl, cycloheptyl, and cyclooctyl. A cycloalkyl group can be unsubstituted or substituted, and may include one or more sites of unsaturation (e.g., cyclopentenyl or cyclohexenyl).

The term "aryl" as used herein refers to a carbocyclic aromatic group. Examples of aryl groups include, but are not limited to, phenyl and naphthyl. An aryl group can be unsubstituted or substituted.

"Heteroaryl" and "heterocycloalkyl" as used herein refer to an aromatic or non-aromatic ring system, respectively, in which one or more ring atoms is a heteroatom, e.g., nitrogen, oxygen, and sulfur. The heteroaryl or heterocycloalkyl group comprises up to 20 carbon atoms and from 1 to 3 heteroatoms selected from N, O, and S. A heteroaryl or heterocycloalkyl may be a monocycle having 3 to 7 ring members (for example, 2 to 6 carbon atoms and 1 to 3 heteroatoms selected from N, O, and S) or a bicycle having 7 to 10 ring members (for example, 4 to 9 carbon atoms and 1 to 3 heteroatoms selected from N, O, and S), for example: a bicyclo[4,5], [5,5], [5,6], or [6,6] system. Examples of heteroaryl groups include by way of example and not limitation, pyridyl, thiazolyl, tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, isoxazolyl, pyrazinyl, pyridazinyl, indolizinyl, isoindolyl, 3H-indolyl, 1H-indazolyl, purinyl, 4H-quinolizinyl, phthalazinyl, naphthyridinyl, quinoxalinyl, quinazolinyl, cinnolinyl, pteridinyl, 4aH-carbazolyl, carbazolyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, benzotriazolyl, benzisoxazolyl, and isatinoyl. Examples of heterocycloalkyls include by way of example and not limitation, dihydroypyridyl, tetrahydropyridyl (piperidyl), tetrahydrothiophenyl, piperidinyl, 4-piperidonyl, pyrrolidinyl, 2-pyrrolidonyl, tetrahydrofuranyl, tetrahydropyranyl, bis-tetrahydropyranyl, tetrahydroquinolinyl, tetrahydroisoquinolinyl, decahydroquinolinyl, octahydroisoquinolinyl, piperazinyl, quinuclidinyl, and morpholinyl. Heteroaryl and heterocycloalkyl groups can be unsubstituted or substituted.

"Substituted" as used herein and as applied to any of the above alkyl, aryl, cycloalkyl, heteroaryl, heterocyclyl, means that one or more hydrogen atoms are each independently replaced with a substituent. Typical substituents include, but are not limited to, -Cl, Br, F, alkyl, -OH, -OCH₃, NH₂, -NHCH₃, - N(CH₃)₂, -CN, -NC(=O)CH₃, -C(=O)-, -C(=O)NH₂, and -C(=O)N(CH₃)₂. Wherever a group is described as "optionally substituted," that group can be substituted with one or more of the above substituents, independently selected for each occasion. In some embodiments, the substituent may be one or more methyl groups or one or more hydroxyl groups.

In some embodiments, the organic acid for ion pairing is an alkyl carboxylic acid. Non-limiting examples of alkyl carboxylic acids include formic acid, acetic acid, propionic acid, butyric acid, valeric acid, caproic acid, heptanoic acid, octanoic acid, nonanoic acid, decanoic acid, undecanoic acid, dodecanoic acid, stearic acid, oleic acid, linoleic acid, linolenic acid, and the like.

In some embodiments, the organic acid for ion pairing is an alkyl sulfonic acid. Non-limiting examples of alkyl sulfonic acids include propanesulfonic acid, heptanesulfonic acid, and octanesulfonic acid.

In some embodiments, the alkyl carboxylic or sulfonic acid is substituted with one or more hydroxyl groups. Non-limiting examples include glycolic acid, 4-hydroxybutyric acid, and lactic acid.

In some embodiments, an organic acid may include more than one carboxylic acid group or more than one sulfonic acid group (e.g., two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like.

In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid, and *p*-toluenesulfonic acid.

Further non-limiting examples of organic acids which may be useful include 2-(4-isobutylphenyl)propanoic acid, 2,2-dichloroacetic acid, 2-hydroxyethanesulfonic acid, 2-oxoglutaric acid, 4-acetamidobenzoic acid, 4-aminosalicylic acid, adipic acid, ascorbic acid (L), aspartic acid (L), alpha-methylbutyric acid, camphoric acid (+), camphor-10-sulfonic acid (+), cinnamic acid, cyclamic acid, dodecylsulfuric acid, ethane-1,2-disulfonic acid, ethanesulfonic acid, furoic acid, galactaric acid, gentisic acid, glucoheptonic acid, gluconic acid, glucuronic acid, glutamic acid, glycerophosphoric acid, glycolic acid, hippuric acid, isobutyric acid, isovaleric acid, lactobionic acid, lauric acid, levulinic acid, malic acid, malonic acid, mandelic acid, methanesulfonic acid, naphthalene-1,5-disulfonic acid, naphthalene-2-sulfonic acid, oleic acid, palmitic acid, pamoic acid, phenylacetic acid, pyroglutamic acid, pyruvic acid, sebacic acid, stearic acid, and undecylenic acid. Examples of suitable organic acids include, but are not limited to, the list of organic acids in **Table 1.**

**Table 1. Non-limiting examples of suitable organic acids**

| **Acid Name** | **log(P)*** |
|---|---|
| benzoic acid | 1.9 |
| phenylacetic | 1.4 |
| p-toluic acid | 2.3 |
| ethyl benzoic acid | 2.9 |
| isopropyl benzoic acid | 3.5 |
| 4-phenylbutyric | 2.4 |
| 2-(4-Isobutylphenyl)propanoic acid | 3.5 |
| 2-napthoxyacetic acid | 2.5 |
| napthylacetic acid | 2.7 |
| heptanoic acid | 2.5 |
| octanoic acid | 3.05 |
| nonanoic acid | 3.5 |
| decanoic acid | 4.09 |
| 9-deceneoic acid | 3.3 |
| 2-deceneoic acid | 3.8 |
| 10-undecenoic acid | 3.9 |
| dodecandioic acid | 3.2 |
| dodecanoic acid | 4.6 |
| myristic acid | 5.3 |
| palmitic acid | 6.4 |
| stearic acid | 7.6 |
| cyclohexanebutanoic acid | 3.4 |
| 1-heptanesulfonic acid | 2.0 |
| 1-octanesulfonic acid | 2.5 |
| 1-nonanesulfonic acid | 3.1 |
| monooctyl succinate | 2.8 |
| tocopherol succinate | 10.2 |
| monomenthyl succinate | 3 |
| monomenthyl glutarate | 3.4 |
| norbixin ((2*E*,4*E*,6*E*,8*E*,10*E*,12*E*,14*E*,16*E*,18*E*)-4,8,13,17-tetramethylicosa-2,4,6,8, 10,12,14,16,18-nonaenedioic acid) | 7.2 |
| bixin ((2E,4E,6E,8E,10E,12E,14E,16Z,18E)-20-methoxy-4,8,13,17-tetramethyl-20-oxoicosa-2,4,6,8,10,12,14,16,18-nonaenoic acid) | 7.5 |

| | |
|---|---|
| *Values obtained from PubChem or calculated | |

The selection of organic acid may further depend on additional properties in addition to consideration of the logP value. For example, an organic acid should be one recognized as safe for human consumption, and which has acceptable flavor, odor, volatility, stability, and the like. Determination of appropriate organic acids is within the purview of one of skill in the art.

In some embodiments, the organic acid is a mono ester of a dicarboxylic acid or a poly-carboxylic acid. In some embodiments, the dicarboxylic acid is malonic acid, succinic acid, glutaric acid, adipic acid, fumaric acid, maleic acid, or a combination thereof. In some embodiments, the dicarboxylic acid is succinic acid, glutaric acid, fumaric acid, maleic acid, or a combination thereof. In some embodiments, the dicarboxylic acid is succinic acid, glutaric acid, or a combination thereof.

In some embodiments, the alcohol forming the mono ester of the dicarboxylic acid is a lipophilic alcohol. Examples of suitable lipophilic alcohols include, but are not limited to, octanol, menthol, and tocopherol. In some embodiments, the organic acid is an octyl mono ester of a dicarboxylic acid, such as monooctyl succinate, monooctyl fumarate, or the like. In some embodiments, the organic acid is a monomenthyl ester of a dicarboxylic acid. Certain menthyl esters may be desirable in oral compositions as described herein by virtue of the cooling sensation they may provide upon use of the product comprising the composition. In some embodiments, the organic acid is monomenthyl succinate, monomenthyl fumarate, monomenthyl glutarate, or a combination thereof. In some embodiments, the organic acid is a monotocopheryl ester of a dicarboxylic acid. Certain tocopheryl esters may be desirable in oral compositions as described herein by virtue of the antioxidant effects they may provide. In some embodiments, the organic acid is tocopheryl succinate, tocopheryl fumarate, tocopheryl glutarate, or a combination thereof.

In some embodiments, the organic acid is a carotenoid derivative having one or more carboxylic acids. Carotenoids are tetraterpenes, meaning that they are produced from 8 isoprene molecules and contain 40 carbon atoms. Accordingly, they are usually lipophilic due to the presence of long unsaturated aliphatic chains, and are generally yellow, orange, or red in color. Certain carotenoid derivatives can be advantageous in oral compositions by virtue of providing both ion pairing and serving as a colorant in the composition. In some embodiments, the organic acid is 2*E*,4*E*,6*E*,8*E*,10*E*,12*E*,14*E*,16*Z*,18*E*)-20-methoxy-4,8,13,17-tetramethyl-20-oxoicosa-2,4,6,8,10,12,14,16,18-nonaenoic acid (bixin) or an isomer thereof. Bixin is an apocarotenoid found in annatto seeds from the achiote tree (*Bixa orellana*) and is the naturally occurring pigment providing the reddish orange color to annatto. Bixin is soluble in fats and alcohols but insoluble in water, and is chemically unstable when isolated, converting via isomerization into the double bond isomer, trans-bixin (β-bixin), having the structure:

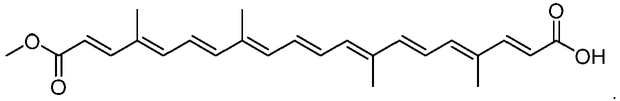

In some embodiments, the organic acid is (2*E*,4*E*,6*E*,8*E*,10*E*,12*E*,14*E*,16*E*,18*E*)-4,8,13,17-tetramethylicosa-2,4,6,8,10,12,14,16,18-nonaenedioic acid (norbixin), a water-soluble hydrolysis product of bixin having the structure:

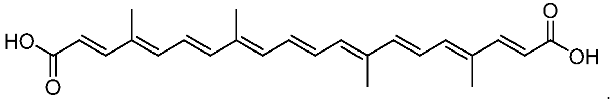

In some embodiments, more than one organic acid may be present. For example, the emulsion may comprise two, or three, or four, or more organic acids for ion pairing. Accordingly, reference herein to "an organic acid" contemplates mixtures of two or more organic acids. The relative amounts of the multiple organic acids may vary. For example, a composition may comprise equal amounts of two, or three, or more organic acids, or may comprise different relative amounts. In this manner, it is possible to include certain organic acids (e.g., citric acid or myristic acid) which have a logP value outside the desired range, when combined with other organic acids to provide the desired average logP range for the combination. In some embodiments, it may be desirable to include organic acids in the emulsion which have logP values outside the desired range for purposes such as, but not limited to, providing desirable organoleptic properties, stability, as flavor components, and the like. Further, certain lipophilic organic acids have undesirable flavor and or aroma characteristics which would preclude their presence as the sole organic acid (e.g., in equimolar or greater quantities relative to nicotine). Without wishing to be bound by theory, it is believed that a combination of different organic acids may provide the desired ion pairing while the concentration of any single organic acid in the emulsion remains below the threshold which would be found objectionable from a sensory perspective.

In some embodiments, the emulsion comprises an organic acid for ion pairing which is a monoester of a dicarboxylic acid or is a carotenoid derivative having one or more carboxylic acids as described herein above, and further comprises an additional organic acid or salt thereof. In some embodiments, the additional organic acid is benzoic acid, an alkali metal salt thereof, or a combination thereof.

In some embodiments, the emulsion comprises an alkali metal salt of an organic acid. For example, at least a portion of the organic acid may be present in the emulsion in the form of an alkali metal salt. Suitable alkali metal salts include lithium, sodium, and potassium. In some embodiments, the alkali metal is sodium or potassium. In some embodiments, the alkali metal is sodium. In some embodiments, the emulsion comprises an organic acid and a sodium salt of the organic acid. In some embodiments, the emulsion comprises benzoic acid, sodium benzoate, or a combination thereof as ion pairing agents.

In some embodiments, the molar ratio of the organic acid to the sodium salt (or other alkali metal) of the organic acid is from about 0.1 to about 10, such as from about 0.1, about 0.25, about 0.3, about 0.5, about 0.75, or about 1, to about 2, about 5, or about 10. For example, in some embodiments, both an organic acid and the sodium salt thereof are added to the other components of the emulsion, wherein the organic acid is added in excess of the sodium salt, in equimolar quantities with the sodium salt, or as a fraction of the sodium salt. One of skill in the art will recognize that the relative amounts will be determined by the desired pH of the emulsion, as well as the desired ionic strength. For example, the organic acid may be added in a quantity to provide a desired pH level of the emulsion, while the alkali metal (e.g., sodium) salt is added in a quantity to provide the desired extent of ion pairing. As one of skill in the art will understand, the quantity of organic acid (i.e., the protonated form) present in the emulsion, relative to the alkali metal salt or conjugate base form present in the composition, will vary according to the pH of the emulsion and the pKa of the organic acid, as well as according to the actual relative quantities initially added to the emulsion.

The amount of organic acid or alkali metal salt thereof present in the emulsion, relative to the active ingredient having the basic amine functionality (e.g., nicotine), may vary. Generally, as the concentration of the organic acid (or the conjugate base thereof) increases, the percent of active ingredient having the basic amine functionality (e.g., nicotine) that is ion paired with the organic acid increases. This typically increases the partitioning of the active ingredient having the basic amine functionality (e.g., nicotine), in the form of an ion pair, into octanol versus water as measured by the logP or logD. In some embodiments, the emulsion comprises from about 0.05, about 0.1, about 1, about 1.5, about 2, or about 5, to about 10, about 15, or about 20 molar equivalents of the organic acid, the alkali metal salt thereof, or the combination thereof, relative to the active ingredient having the basic amine functionality (e.g., nicotine), calculated as the free base of the active ingredient having the basic amine functionality (e.g., nicotine).

In some embodiments, the emulsion comprises from about 2 to about 10, or from about 2 to about 5 molar equivalents of the organic acid, the alkali metal salt thereof, or the combination thereof, relative to the s active ingredient having the basic amine functionality (e.g., nicotine), on a free-base basis. In some embodiments, the organic acid, the alkali metal salt thereof, or the combination thereof, is present in a molar ratio with the active ingredient having the basic amine functionality (e.g., nicotine) from about 2, about 3, about 4, or about 5, to about 6, about 7, about 8, about 9, or about 10. In embodiments wherein more than one organic acid, alkali metal salt thereof, or both, are present, it is to be understood that such molar ratios reflect the totality of the organic acids present.

In certain embodiments the organic acid inclusion is sufficient to provide an emulsion pH of from about 4.0 to about 9.0, such as from about 4.5 to about 7.0, or from about 5.5 to about 7.0, from about 4.0 to about 5.5, or from about 7.0 to about 9.0. In some embodiments, the organic acid inclusion is sufficient to provide a n emulsion pH of from about 4.5 to about 6.5, for example, from about 4.5, about 5.0, or about 5.5, to about 6.0, or about 6.5. In some embodiments, the organic acid is provided in a quantity sufficient to provide a pH of the emulsion of from about 5.5 to about 6.5, for example, from about 5.5, about 5.6, about 5.7, about 5.8, about 5.9, or about 6.0, to about 6.1, about 6.2, about 6.3, about 6.4, or about 6.5. In other embodiments, a mineral acid (e.g., hydrochloric acid, sulfuric acid, phosphoric acid, or the like) or a buffer as described herein is added to adjust the pH of the emulsion to the desired value.

### Solvent

The emulsion comprises one or more solvents. In some embodiments, the solvent is water. In some embodiments, a portion, or even all of the water in the emulsion may be replaced with a humectant. Suitable humectants include, but are not limited to, polyols such as propylene glycol, glycerol, 1,2-propanediol, and the like, including combinations thereof. Such humectants may be referred to as cosolvents and may also serve to stabilize the emulsion and/or reduce the amount of emulsifying agent required to form a stable emulsion.

The amount of solvent in the emulsion may vary according to the desired properties of the emulsion and the nature and relative quantities of the other emulsion components present (e.g., active ingredient, flavorant, emulsifier). In some embodiments, the solvent is water, and the water content of the emulsion is from about 65% by weight to about 70% by weight, based on the total weight of the emulsion. In some embodiments, the emulsion comprises greater than about 60% by weight of water, and less than about 40% by weight of the SEDS component.

### Buffering Agent

The emulsion generally comprises a buffering agent. Examples of suitable buffering agents include, but are not limited to, alkali metal carbonates (e.g., potassium carbonate or sodium carbonate), bicarbonates, such as sodium bicarbonate, alkali metals acetates, glycinates, phosphates, glycerophosphates, citrates, borates, or mixtures thereof. In some embodiments, the buffering agent is sodium carbonate, sodium bicarbonate, potassium carbonate, potassium bicarbonate, or a mixture thereof. In some embodiments, the buffering agent is sodium bicarbonate.

The buffering agent is typically present in an amount less than about 5% based on the total weight of the composition; for example, from about 0.1% to about 5%, such as, e.g., from about 0.1% to about 1%, or from about 0.1% to about 0.5%, or from about 0.1% to about 0.3% by weight, based on the total weight of the composition.

### Properties of the Emulsion

As described herein above, the composition comprises an oil-in-water emulsion. Without wishing to be bound by theory, it is believed that the emulsion serves to protect the active ingredient, flavorant, or both from degradation or premature volatilization, and may aid in providing more rapid and/or complete delivery of the active ingredient, flavorant, or both to the consumer. In some embodiments, the emulsion can be characterized as a liquid or a viscous gel. In some embodiments, the emulsion has a viscosity in a range from about 0.001 to about 0.25 Pascals at a shear rate from about 0.1 to about 300 s⁻¹.

In some embodiments, the emulsion comprises particles having an average size (i.e., diameter) in a range from about 10 nm to about 1,000 nm. "Average particle size" is synonymous with D₅₀, meaning half of the population of particles has a particle size above this point, and half below. D₉₀ particle size distribution indicates that 90% of the particles (by number) have a Feret diameter below a certain size. The size of nanoparticles may be determined by quasi-electric light scattering (QELS) as described in Bloomfield, Ann. Rev. Biophys. Bioeng., 10:421-450 (1981), incorporated herein by reference. It may also be measured by correlation spectroscopy that analyzes the fluctuation in scattering of light due to Brownian motion, or by transmission electron microscopy (TEM). Unless otherwise indicated, particle size values reported herein are determined by light scattering using a nanoscale Zetasizer (Malvern Zetasizer; Malvern Panalytical Inc., 117 Flanders Road, Westborough, MA, USA).

In some embodiments, the D₅₀ particle size is from about 100 nm to about 300 nm. In some embodiments, the D₅₀ particle size is about 100 nm, about 110 nm, about 120 nm, about 130 nm, about 140 nm, about 150 nm, about 160 nm, about 170 nm, about 180 nm, about 190 nm, about 200 nm, about 210 nm, about 220 nm, about 230 nm, about 240 nm, about 250 nm, about 260 nm, about 270 nm, about 280 nm, about 290 nm, or about 300 nm. Without wishing to be bound by theory, it is believed that droplets in this size range may be more readily absorbed through the intestinal lumen, may be available for lymphatic distribution, or both.

The emulsion as described herein may be characterized by reference to a polydispersity index. Polydispersity indicates the uniformity of droplet size in an emulsion. The higher the value of polydispersity, the lower will be the uniformity of droplet size. It may be defined as the ratio of standard deviation to mean droplet size and may be measured by spectrophotometric methods. In some embodiments, the emulsion has a polydispersity index of less than about 0.5.

The emulsion as described herein may be characterized by reference to zeta potential. As appreciated by one skilled in the art, zeta potential is the measure of the electrical charge on particle surface in colloidal dispersions, for example, the charge on the surface of a droplet in the emulsion. Zeta potential may be measured with a zeta analyzer, for example a Malvern nano-series ZS Zetasizer. In some embodiments, the zeta potential of the particles is from about -50 mV to about +50 mV. In some embodiments, the zeta potential of the particles is from about -50 mV, about -40 mV, about -30 mV, about -20 mV, about -10 mV, or about 0 mV, to about 10 mV, about 20 mV, about 30 mV, about 40 mV, or about 50 mV.

### Composition

In another aspect is provided a composition comprising an emulsion as described herein above. The composition comprises the emulsion, one or more fillers, and optionally, one or more sweeteners; one or more salts, and one or more buffering agents.

### Filler

The composition as disclosed herein comprises one or more fillers. Fillers may fulfill multiple functions, such as enhancing certain organoleptic properties such as texture and mouthfeel, enhancing cohesiveness or compressibility of the product, and the like. In some cases, fillers can serve multiple functions, and therefore some components of the composition can be considered both a filler and, for example, a flow aid, a sweetener, or a binder. Generally, fillers are porous particulate materials and are cellulose-based. For example, suitable fillers are any non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX^{®} brand filler available from International Fiber Corporation), bran fiber, and mixtures thereof. Non-limiting examples of derivatives of non-tobacco plant material include starches as described herein (e.g., from potato, wheat, rice, corn), natural cellulose, and modified cellulosic materials.

In some embodiments, the filler includes a non-tobacco plant material or a derivative thereof. Non-limiting examples of derivatives of non-tobacco plant material include starches (e.g., from potato, wheat, rice, corn), natural cellulose, and modified cellulosic materials. "Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the oral product based on the ability of the starch material to impart a specific organoleptic property to the oral product. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications and are considered to be "genetically modified" starches. Other starches are obtained and subsequently modified by chemical, enzymatic, or physical means. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, acetylation, hydroxypropylation, and/or partial hydrolysis. Enzymatic treatment includes subjecting native starches to enzyme isolates or concentrates, microbial enzymes, and/or enzymes native to plant materials, e.g., amylase present in corn kernels to modify corn starch. Other starches are modified by heat treatments, such as pregelatinization, dextrinization, and/or cold-water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, and starch sodium octenyl succinate.

Additional examples of potential fillers include maltodextrin, dextrose, lactose, calcium carbonate, calcium phosphate, sugar alcohols, and cellulosic materials. Combinations of fillers can also be used.

In some embodiments, the filler comprises one or more sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). In some embodiments, the composition is substantially free of sugar alcohols. In some embodiments, the composition is substantially or completely free of xylitol or other sugar alcohols. In some embodiments, the presence of sugar alcohols such as xylitol can consume the continuous phase of the emulsion, resulting in phase inversion and loss of the emulsion. In some embodiments, any sugar alcohol is added during final composition blending (e.g., immediately before pouching and optional overspray with water) to minimize impact on the emulsion.

In some embodiments, the filler comprises a cellulose material or a cellulose derivative, such as microcrystalline cellulose ("mcc"). The mcc may be synthetic or semi-synthetic, or it may be obtained entirely from natural celluloses. The mcc may be selected from the group consisting of AVICEL^{®} grades PH-100, PH-102, PH-103, PH-105, PH-112, PH-113, PH-200, PH-300, PH-302, VIVACEL^{®} grades 101, 102, 12, 20 and EMOCEL^{®} grades 50M and 90M, and the like, and mixtures thereof.

In some embodiments, the filler comprises or is an inorganic material. Examples of potential inorganic fillers include calcium carbonate, calcium phosphate, and bioceramic materials (e.g., porous hydroxyapatite).

In some embodiments, a particulate filler can be characterized as substantially spherical, such as cellulose spheres. By "substantially spherical" is meant at least a portion of the particulate filler component is in the shape of a sphere and/or is "sphere-like' in shape. As such, "substantially spherical" encompasses slightly elongated (e.g., oval) shapes, slightly flattened shapes, and the like. Substantially spherical particulate filler components are intended to be distinguished from conventional particulate filler components (e.g., commercially available "fillers" or "particulate fillers" that are not explicitly designed as "spherical"). Such conventional particulate filler components typically substantially comprise particles with rather irregular shapes. In some cases, at least some (e.g., including a majority) of such conventional particulate filler components comprise one or more edges (e.g., jagged edges) that are typically not observable on substantially spherical particulate filler components as employed in the context of the present disclosure. Further, the uniformity of the particle shapes and sizes of a substantially spherical filler component is generally much greater than that of a conventional particulate filler component.

In some embodiments, the substantially spherical filler component comprises MCC. In some embodiments, the substantially spherical filler component comprises solid (although porous) MCC spheres. In some embodiments, the substantially spherical filler component comprises hollow MCC spheres. In some embodiments, the center/core of such hollow MCC spheres may be unfilled; in some embodiments, the center/core of such hollow MCC spheres may be filled with one or more additional components (e.g., flavorants, fillers, active ingredients, etc.). Examples of suitable MCC spheres include, but are not limited to, Vivapur^{®} MCC spheres from JRS Pharma, available, e.g., with particle sizes of 100-200 µm (Vivapur^{®} 100), 200-355 µm (Vivapur^{®} 200), 355-500 µm (Vivapur^{®} 350), 500-710 µm (Vivapur^{®} 500), 710-1000 µm (Vivapur^{®}700), and 1000-1400 µm (Vivapur^{®} 1000). Further examples of suitable MCC spheres include, but are not limited to, Celphere^{™} MCC spheres from Asahi Kasei Corporation, available, e.g., with particle sizes of 75-212 µm (Celphere^{™} SCP-100), 106-212 µm (Celphere^{™} CP-102), 150-300 µm (Celphere^{™} CP-203), 300-500 µm (Celphere^{™} CP-305), and 500-710 µm (Celphere^{™} CP-507).

The average diameter of the substantially spherical particulate filler particles provided herein can vary and is not particularly limited. For example, in some embodiments, the spherical filler particles have an average diameter of about 100 microns to about 1000 microns, such as about 250 microns to about 750 microns. For example, in some embodiments, the average diameter is about 100 microns to about 500 microns, e.g., about 100 microns to about 400 microns, about 100 microns to about 300 microns, about 100 microns to about 200 microns, about 200 microns to about 500 microns, about 200 microns to about 400 microns, about 200 microns to about 300 microns, about 300 microns to about 500 microns, about 300 microns to about 400 microns, or about 400 microns to about 500 microns. In some embodiments, the average diameter is about 500 microns to about 1000 microns, e.g., about 500 microns to about 900 microns, about 500 microns to about 800 microns, about 500 microns to about 700 microns, about 500 microns to about 600 microns, about 600 microns to about 1000 microns, about 600 microns to about 900 microns, about 600 microns to about 800 microns, about 600 microns to about 700 microns, about 700 microns to about 1000 microns, about 700 microns to about 900 microns, about 700 microns to about 800 microns, about 800 microns to about 1000 microns, about 800 microns to about 900 microns, or about 900 microns to about 1000 microns.

The distribution of diameters around this average diameter (i.e., the particle size distribution) can also vary; in some embodiments, the distribution of diameters is close to the listed value (e.g., +/- about 25% of the stated value, +/- about 20% of the stated value, +/- about 15% of the stated value, +/- about 10% of the stated value, +/- about 5% of the stated value, or +/- about 1% of the stated value. The disclosure is not, however, limited to materials with such narrow distributions; in some embodiments, the diameter of the MCC spheres within a given material can vary within a wider range.

The amount of filler can vary, but is typically greater than about 10%, and up to about 75% of the composition by weight, based on the total weight of the composition. A typical range of filler within the composition can be from about 10 to about 75% by total weight of the composition, for example, from about 10, about 15, about 20, about 25, or about 30, to about 35, about 40, about 45, or about 50% by weight (e.g., about 10 to about 50%, or about 25 to about 45% by weight). In some embodiments, the amount of filler is at least about 20% by weight, such as at least about 25%, or at least about 30%, or at least about 35%, or at least about 40%, based on the total weight of the composition. In some embodiments, the amount of filler within the composition can be from about 32 to about 48% by total weight of the composition.

In some embodiments, at least a portion of the emulsion is adsorbed in or on the filler.

### Sweetener

In order to improve the sensory and/or physical properties of the composition, one or more sweeteners may be added. The sweeteners can be any sweetener or combination of sweeteners, in natural or artificial form, or as a combination of natural and artificial sweeteners. Examples of natural sweeteners include fructose, sucrose, glucose, maltose, isomaltulose, mannose, galactose, lactose, stevia, honey, and the like. Examples of artificial sweeteners include sucralose, maltodextrin, saccharin, aspartame, acesulfame K, neotame and the like. In some embodiments, the sweetener is selected from the group consisting of fructose, sucrose, glucose, maltose, mannose, galactose, lactose, stevia, honey, sucralose, isomaltulose, maltodextrin, saccharin, aspartame, acesulfame K, neotame, erythritol, arabitol, ribitol, isomalt, maltitol, dulcitol, iditol, mannitol, xylitol, lactitol, sorbitol, and mixtures thereof. In some embodiments, the sweetener is selected from the group consisting of acesulfame K, aspartame, sucralose, and combinations thereof.

When present, the sweetener or mixture of sweeteners may be present in an amount from about 0.1% to about 10% by weight of the composition, such as about 1 to about 5%, or about 1 to about 3% by weight, based on the total weight of the composition.

### Salt

In some embodiments, the composition comprises a salt (e.g., an alkali metal salt), typically employed in an amount sufficient to provide desired sensory attributes to the product. Non-limiting examples of suitable salts include sodium chloride, potassium chloride, ammonium chloride, flour salt, sodium acetate, sodium citrate, and the like.

When present, a representative amount of salt is at least about 0.5% by weight, such as at least about 1% by weight, such as at least about 1.5% by weight. In some embodiments, the composition may comprise a salt in an amount of from about 0.5% to about 10% by weight, such as from about 1% to about 7.5% by weight, such as from about 1.5% to about 5% by weight, or from about 2 to about 3% by weight, based on the total weight of the composition.

### Water

In some embodiments, the composition, or the overall oral product comprising such composition, comprises water. The water may be present in the emulsion (e.g., as part or all of the solvent), may be present solely as a component of the composition or product, or may be present in both the emulsion and the composition or product as a whole (i.e., in some embodiments, the composition and/or the oral product comprises water as part of the emulsion, and also comprises a further amount of water beyond that present in the emulsion). The amount of water present in the overall composition will vary depending upon the desired characteristics of the specific composition and the format thereof. In some embodiments, the composition comprises water in an amount from about 15 to about 25% by weight, based on the total weight of the composition.

In some embodiments, a pouched oral product comprising the composition may comprise about 5% to about 60% water by weight, such as from about 5, about 10, about 15, about 20, or about 25%, to about 30, about 35, about 40, about 45, about 50, about 55, or about 60% water by weight, based on the total weight of the pouched product. In some embodiments, a pouched oral product comprising the composition may comprise about 40 to about 60% water by weight, or about 40 to about 50% water by weight, based on the total weight of the pouched product. In some embodiments, the pouched oral product comprising the composition comprises about 40, about 45, about 50, about 55, or about 60% water by weight, based on the total weight of the pouched product.

### Humectant

In some embodiments, one or more humectants may be employed in the composition, and may be present in the emulsion as described above, or may be in the overall composition, or in both the emulsion and the overall composition. Examples of humectants include, but are not limited to, glycerin, 1,2-propanediol (propylene glycol), 1,3-propanediol, dipropylene glycol, sorbitol, xylitol, mannitol, and the like. In some embodiments, the emulsion, the composition, or both are substantially free of humectants.

Where included, the humectant is typically provided in an amount sufficient to provide desired moisture attributes to the composition. When present, the humectant (such as glycerin and/or propylene glycol) may be present in an amount of from about 5% to about 60% by weight, such as from about 5, about 10, about 15, about 20, or about 25%, to about 30, about 35, about 40, about 45, about 50, about 55, or about 60% humectant by weight, based on the total weight of the pouched product. In some embodiments, a pouched oral product comprising the composition may comprise about 0.1% to about 15% by weight of the composition, such as from about 1 to about 12%, or from about 7 to about 11% by weight of the pouched product.

### Moisture Content

The moisture content of the composition may vary, as may that of a pouched product comprising such composition. As used herein, the term "moisture content" refers to a total amount by weight in the composition or pouched oral product of water, humectant, or a combination thereof. For example, a composition or pouched product may include water, or may include one or more humectants, or may include both water and a humectant, each as described herein above, and in the quantities described herein above. In some embodiments, the moisture content of a pouched oral product of the disclosure is in a range from about 5 to about 60% by weight, such as from about 5, about 10, about 15, about 20, or about 25%, to about 30, about 35, about 40, about 45, about 50, about 55, or about 60%, based on the total weight of the pouched product. In some embodiments, the entirety of the moisture content is provided by water. In some embodiments, the entirety of the moisture content is provided by one or more humectants. In some embodiments, the moisture content is provided by a mixture of water and humectant.
In some embodiments, the moisture content can be determined by analytical methods. For example, determination of water content can be performed using the Karl Fischer test as outlined in ISO 760:1978. Moisture content, reflecting both water and humectant, may be determined as oven volatiles. Oven volatiles are defined as the reduction in mass when a sample is dried in a forced draft oven at a temperature regulated to 100°C ± 1 °C for three hours ± 0.5 minutes. The determination of moisture content as oven volatiles reflects all volatile constituents, such as water, humectant, flavoring components, and the like that are lost under the specified conditions. Accordingly, moisture content reported as oven volatiles may be higher than the total amount by weight of water and humectant in the presence of other volatile components in the composition. However, oven volatile content is likely to be within the same general ranges provided above for moisture content.

### Colorants

A colorant may be employed in amounts sufficient to provide the desired physical attributes to the oral product. Natural or synthetic colorants, such as natural or synthetic dyes, food-grade colorants and pharmaceutical-grade colorants may be used. Examples of colorants include various dyes and pigments, such as caramel coloring and titanium dioxide. Natural colorants such as curcumin, beet juice extract, spirulina may be used. Also, a variety of synthetic pigments may be used. In some embodiments, the colorant is a lake dye, such as a red or blue aluminum lake dye. The amount of colorant utilized in the oral product can vary, but when present is typically up to about 3% by weight, such as from about 0.1%, about 0.5%, or about 1%, to about 3% by weight, based on the total weight of the composition.

### Antioxidants

In some embodiments, the composition comprises an antioxidant or a combination of antioxidants. As used herein, the term "antioxidant" refers to a substance which prevents or suppresses oxidation by terminating free radical reactions and may delay or prevent degradation of a composition component (e.g., flavor component, active ingredient, or lipid component). Antioxidants may be naturally occurring or synthetic. Naturally occurring antioxidants include those found in foods and botanical materials. Non-limiting examples of antioxidants include certain botanical materials, vitamins, polyphenols, phenol derivatives, citric acid, Vitamin E or a derivative thereof, vitamin C or a derivative thereof (including fatty acid esters), a tocopherol, epicatechol, epigallocatechol, epigallocatechol gallate, erythorbic acid, sodium erythorbate, 4-hexylresorcinol, theaflavin, theaflavin monogallate A or B, theaflavin digallate, phenolic acids, glycosides, quercitrin, isoquercitrin, hyperoside, polyphenols, catechols, resveratrols, oleuropein, hydroquinone, butylated hydroxyanisole (BHA), butylated hydroxytoluene (BHT), tertiary butylhydroquinones (TBHQs), such as mono- tertiary butylhydroquinone (MTBHQ) and di-tertiary butylhydroquinone (DTBHQ), sodium benzoate, and combinations thereof. Typically, the antioxidant(s) will be lipophilic. In some embodiments, the antioxidant is a tocopherol, BHT, TBHQ, vitamin E or a derivative thereof.

When present, an antioxidant is typically at a concentration of from about 0.001% w/w to about 10% by weight, such as, e.g., from about from about 0.001%, about 0.005%, about 0.01% w/w, about 0.05%, about 0.1%, or about 0.5%, to about 1%, about 2%, about 3%, about 4%, about 5%, about 6%, about 7%, about 8%, about 9%, or about 10%, based on the total weight of the composition. In some embodiments, the emulsion or the overall composition comprises one or more antioxidants in a total amount from about 0.1 to about 1% by weight, such as from about 0.4 to about 0.6% by weight.

### Oral care additives

In some embodiments, the composition comprises an oral care ingredient (or mixture of such ingredients). Oral care ingredients provide the ability to inhibit tooth decay or loss, inhibit gum disease, relieve mouth pain, whiten teeth, or otherwise inhibit tooth staining, elicit salivary stimulation, inhibit breath malodor, freshen breath, or the like. For example, effective amounts of ingredients such as thyme oil, eucalyptus oil and zinc (e.g., such as the ingredients of formulations commercially available as ZYTEX^{®} from Discus Dental) can be incorporated into the composition. Other examples of ingredients that can be incorporated in desired effective amounts within the present composition can include those that are incorporated within the types of oral care compositions set forth in Takahashi et al., Oral Microbiology and Immunology, 19(1), 61-64 (2004); U.S. Pat. No. 6,083,527 to Thistle; and US Pat. Appl. Pub. Nos. 2006/0210488 to Jakubowski and 2006/02228308 to Cummins et al. Other exemplary ingredients include those contained in formulations marketed as MALTISORB^{®} by Roquette and DENTIZYME^{®} by NatraRx. When present, a representative amount of oral care additive is at least about 1%, often at least about 3%, and frequently at least about 5% of the total dry weight of the composition. The amount of oral care additive within the composition will not typically exceed about 30%, often will not exceed about 25%, and frequently will not exceed about 20%, of the total dry weight of the composition.

### Other Additives

Other additives can be included in the composition. For example, the composition can be processed, blended, formulated, combined, and/or mixed with other materials or ingredients. The additives can be artificial or can be obtained or derived from herbal or biological sources. Examples of further types of additives include tobacco (including whitened tobacco), thickening or gelling agents (e.g., fish gelatin, alginates), preservatives (e.g., potassium sorbate, sodium benzoate, calcium propionate, and the like), disintegration aids, zinc or magnesium salts selected to be relatively water soluble for oral products with greater water solubility (e.g., magnesium or zinc gluconate) or selected to be relatively water insoluble for oral products with reduced water solubility (e.g., magnesium or zinc oxide), or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No. 2007/0062549 to Holton, Jr. et al., each of which is incorporated herein by reference.

Typical inclusion ranges for such additional additives can vary depending on the nature and function of the additive and the intended effect on the final composition, with an example range of up to about 10% by weight, (e.g., from about 0.1% to about 5% by weight) based on total weight of the composition. In some embodiments, the composition is substantially or completely free of alginates. In some embodiments, the presence of alginate can consume the continuous phase of the emulsion, resulting in phase inversion and loss of the emulsion. In some embodiments, any alginate is added during final composition blending (e.g., immediately before pouching and optional overspray with water) to minimize impact on the emulsion. In some embodiments, the composition is substantially or completely free of tobacco material (exclusive of nicotine content, if present).

### Configured for Oral Use

The composition as described herein are configured for oral use. The term "configured for oral use" as used herein means that the composition is provided in a form such that during use, saliva in the mouth of the user causes one or more of the components of the composition (e.g., flavoring agents and/or active ingredients) to pass into the mouth of the user. In some embodiments, the composition is adapted to deliver components to a user through mucous membranes in the user's mouth, the user's digestive system, or both, and, in some instances, said component is an active ingredient that can be absorbed through the mucous membranes in the mouth or absorbed through the digestive tract when the product is used.. Compositions configured for oral use as described herein may take various forms, including gels, pastilles, gums, lozenges, pellets, tablets, granules, beads, powders, and pouches. Gels can be soft or hard. Certain products configured for oral use are in the form of pastilles. As used herein, the term "pastille" refers to a dissolvable oral product made by solidifying a liquid or gel composition so that the final product is a somewhat hardened solid gel. The rigidity of the gel is highly variable. Certain products of the disclosure are in the form of solids. Certain products can exhibit, for example, one or more of the following characteristics: crispy, granular, chewy, syrupy, pasty, fluffy, smooth, and/or creamy. In certain embodiments, the desired textural property can be selected from the group consisting of adhesiveness, cohesiveness, density, dryness, fracturability, graininess, gumminess, hardness, heaviness, moisture absorption, moisture release, mouthcoating, roughness, slipperiness, smoothness, viscosity, wetness, and combinations thereof.

The products comprising the compositions of the present disclosure may be dissolvable. As used herein, the terms "dissolve," "dissolving," and "dissolvable" refer to compositions having aqueous-soluble components that interact with moisture in the oral cavity and enter into solution, thereby causing gradual consumption of the product. According to one aspect, the dissolvable product is capable of lasting in the user's mouth for a given period of time until it completely dissolves. Dissolution rates can vary over a wide range, from about 1 minute or less to about 60 minutes. For example, fast release compositions typically dissolve and/or release the active substance in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the composition. In some embodiments, the product can be meltable as discussed, for example, in US Patent App. Pub. No. 2012/0037175 to Cantrell et al. In other embodiments, the products do not dissolve during the product's residence in the user's mouth.

The compositions as disclosed herein can be formed into a variety of shapes, including pills, tablets, spheres, beads, ovoids, obloids, and the like. Cross-sectional shapes of the composition can vary, and example cross-sectional shapes include circles, squares, ovals, rectangles, and the like. Such shapes can be formed in a variety of manners using equipment such as moving belts, nips, extruders, granulation devices, compaction devices, and the like.

In some embodiments, compositions configured for oral use as described herein comprise the composition of the present disclosure disposed within a moisture-permeable container (e.g., a water-permeable pouch). The composition may take various forms, including pellets, tablets, granules, beads, and powders disposed within the moisture-permeable container. In some embodiments, the composition is in particulate form (e.g., powder or granular) enclosed within a pouch. Such compositions in the water-permeable pouch format are typically used by placing one pouch containing the composition in the mouth of a human subject/user. Generally, the pouch is placed somewhere in the oral cavity of the user, for example under the lips, in the same way as moist snuff products are generally used. The pouch generally is not chewed or swallowed. Exposure to saliva then causes some of the components of the emulsion (e.g., flavoring agents and/or active ingredients) to pass through e.g., the water-permeable pouch and provide the user with flavor and satisfaction. After about 5 minutes to about 60 minutes, typically about 10 minutes to about 45 minutes of use/enjoyment, substantial amounts of the desired components have been released and absorbed through oral mucosa of the human subject, and the pouch may be removed from the mouth of the human subject for disposal.

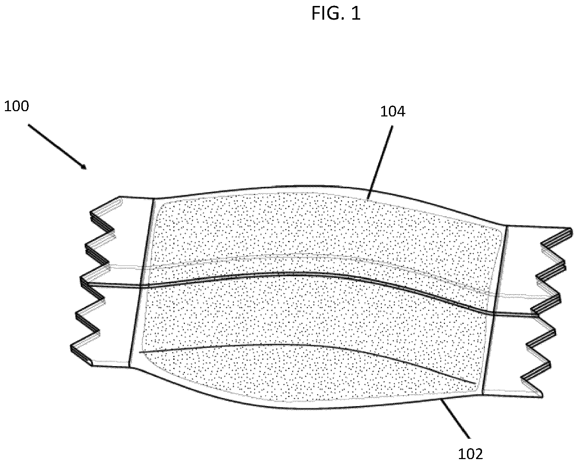

Some embodiments of the disclosure will be described with reference to the accompanying drawing, and these described embodiments involve snus-type products having an outer pouch and containing a composition as described herein. As explained in greater detail below, such embodiments are provided by way of example only, and the pouched products of the present disclosure can include the composition in other forms. Referring to **FIG. 1**, there is shown a first embodiment of a pouched product **100**. The pouched product **100** includes a moisture-permeable container in the form of a pouch **102**, which contains a composition **104** as described herein. Pouches as described herein have three dimensions: a length, a width, and a thickness. One of skill in the art will recognize that such dimensions may vary depending on the intended overall size and volume of the pouch, and the quantity of material desired within the pouch.

The type of pouch may vary. The pouch provides a liquid-permeable container of a type that may be considered to be similar in character to the mesh-like type of material that is used for the construction of a tea bag. Components of the composition readily diffuse through the pouch and into the mouth of the user.

Non-limiting examples of suitable types of pouches are set forth in, for example, US Pat. Nos. 5,167,244 to Kjerstad and 8,931,493 to Sebastian et al.; as well as US Patent App. Pub. Nos. 2016/0000140 to Sebastian et al.; 2016/0073689 to Sebastian et al.; 2016/0157515 to Chapman et al.; and 2016/0192703 to Sebastian et al., each of which is incorporated herein by reference. Pouches can be provided as individual pouches, or a plurality of pouches (e.g., 2, 4, 5, 10, 12, 15, 20, 25 or 30 pouches) can be connected or linked together (e.g., in an end-to-end manner) such that a single pouch or individual portion can be readily removed for use from a one-piece strand or matrix of pouches.

The pouches of the present disclosure can be formed from a fleece material, e.g., fibrous nonwoven webs. As used herein, the term "fiber" is defined as a basic element of textiles. Fibers are often in the form of a rope- or string-like element. As used herein, the term "fiber" is intended to include fibers, filaments, continuous filaments, staple fibers, and the like, which can be either monocomponent or multicomponent. The term "multicomponent fibers" refers to fibers that comprise two or more components that are different by physical or chemical nature, including bicomponent fibers. Specifically, the term "multicomponent fibers" includes staple and continuous fibers prepared from two or more polymers present in discrete structured domains in the fiber, as opposed to blends where the domains tend to be dispersed, random or unstructured.

A "fleece material" as used herein may be formed from various types of fibers (e.g., cellulosic fibers; such as viscose fibers, regenerated cellulose fibers, cellulose fibers, and wood pulps; cotton fibers; other natural fibers; or synthetic fibers) capable of being formed into a traditional fleece fabrics or other traditional pouch materials. For example, fleece materials may be provided in the form of a woven or nonwoven fabric. Suitable types of fleece materials, for example, are described in U.S. Patent No. 8,931,493 to Sebastian et al.; US Patent App. Pub. No. 2016/0000140 to Sebastian et al.; and US Patent App. Pub. No. 2016/0073689 to Sebastian et al.; which are all incorporated herein by reference.

The term "nonwoven" is used herein in reference to fibrous materials, webs, mats, batts, or sheets in which fibers are aligned in an undefined or random orientation. The nonwoven fibers are initially presented as unbound fibers or filaments. An important step in the manufacturing of nonwovens involves binding the various fibers or filaments together. The manner in which the fibers or filaments are bound can vary, and include thermal, mechanical and chemical techniques that are selected in part based on the desired characteristics of the final product, as discussed in more detail herein below.

In some embodiments, the pouch may be biodegradable or dissolvable. In some embodiments, after use, the entire composition, and in some embodiments, the entirety of the pouch material originally housing the composition, can be dissolved and orally ingested by the user such that there is nothing left of the pouched product to remove from the mouth of the user. An example dissolvable pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and composition each may undergo complete dispersion within the mouth of the user during normal conditions of use, and hence the pouch and composition both may be ingested by the user. In various embodiments, the pouch material can be dissolvable (i.e., orally ingestible) such that under conditions of normal use (i.e., upon contact with saliva in the mouth of a user), the pouch material completely dissolves. Typically, the pouch material will dissolve after a significant amount of the soluble components of the composition within the pouch (e.g., active ingredient(s) and/or flavorant(s)) permeate through the pouch material into the mouth of the user. For example, the pouch material can be configured to dissolve at a rate such that the pouch material holds the composition together for a period of time sufficient to allow for the release of substantially all water-soluble components.

A pouched product as described herein can be packaged within any suitable inner packaging material and/or outer container. See also, for example, the various types of containers set forth in US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al. which are incorporated herein by reference.

The amount of material contained within each pouch may vary. In some embodiments, the weight of the composition within each pouch is at least about 50 mg, for example, from about 50 mg to about 2 grams, from about 100 mg to about 1.5 grams, or from about 200 to about 700 mg. In some smaller embodiments, the weight of the composition within each pouch may be from about 100 to about 300 mg. For a larger embodiment, the weight of the material within each pouch may be from about 300 mg to about 700 mg.

If desired, other components can be contained within each pouch. For example, at least one flavored strip, piece or sheet of flavored water dispersible or water-soluble material (e.g., a breath-freshening edible film type of material) may be disposed within each pouch along with or without at least one capsule. Such strips or sheets may be folded or crumpled in order to be readily incorporated within the pouch. See, for example, the types of materials and technologies set forth in US Pat. Nos. 6,887,307 to Scott et al. and 6,923,981 to Leung et al.; and The EFSA Journal (2004) 85, 1-32; which are incorporated herein by reference. In some embodiments, the pouch contents further comprise microcrystalline cellulose as described herein above.

While the compositions described herein have generally been described as disposed within a pouch, products of the present disclosure are not limited to such pouched embodiments. Accordingly, any embodiment of the disclosed composition may also be used in the absence of a pouch. For instance, a composition as described herein may simply be applied directly to the oral cavity as e.g., a powder, granules, beads, tablets, pellets, or the like.

### Preparation of the composition

In a general, non-limiting embodiment, compositions of the disclosure are prepared by first preparing an emulsion, then combining the emulsion with other components to form the composition. In some embodiments, an emulsifying agent, a lipid component, a flavoring agent, and an active ingredient each as described herein above, are combined to form a self-emulsifying delivery system (SEDS) component (also referred to herein as a "pre-emulsion"). In some embodiments, the flavoring agent is dissolved in the lipid component prior to combination with the active ingredient and emulsifying agent. In some embodiments, the flavoring agent is obtained as a solution in a suitable lipid, such as MCT oil. The SEDS components may be contacted, combined, or mixed together using any mixing technique or equipment known in the art. Any mixing method that brings the oral product ingredients into intimate contact can be used, such as a mixing apparatus featuring an impeller or other structure capable of agitation. Examples of mixing equipment include casing drums, conditioning cylinders or drums, liquid spray apparatus, conical-type blenders, ribbon blenders, mixers available as FKM130, FKM600, FKM1200, FKM2000 and FKM3000 (Littleford Day, Inc.), Plough Share types of mixer cylinders, Hobart mixers, and the like. See also, for example, the types of methodologies set forth in US Pat. Nos. 4,148,325 to Solomon et al.; 6,510,855 to Korte et al.; and 6,834,654 to Williams, each of which is incorporated herein by reference. In some embodiments, the combining is performed by mixing the components by stirring. In some embodiments, the emulsifying agent, lipid component, flavoring, and active ingredient are stirred together for a period of time at a speed in a range from about 100 to about 1000 rpm.

The SEDS component is then combined with a solution comprising a buffering agent and water to form an emulsion (e.g., an oil-in-water emulsion). The nature and quantity of the buffering agent and quantity of water are as described herein above. In some embodiments, at least a portion of the water is replaced with a humectant as described herein above. In some embodiments, the combining is performed by stirring. In some embodiments, the SEDS and buffer solution are stirred together for a period of time at a speed in a range from about 100 to about 1000 rpm to form the oil-in-water emulsion.

Surprisingly, it has been found according to the present disclosure that the order of combining the various components is affects the stability and properties of the emulsion. Further, the quantity of water utilized and the ratio of emulsifying agent to lipid component is an important factor in the stability and properties of the emulsion. For example, as discussed in Examples 1-4 herein below, clarity and stability of the emulsions was dependent upon the order in which the components were combined. Specifically, adding a mixture of lipid component, flavoring, and surfactant to active, buffer, and water; adding a mixture of lipid component, flavoring, and surfactant to active and water, followed by adding buffer; and adding a mixture of lipid component, flavoring, surfactant, and active to water and adding buffer all resulted in emulsions with varying degrees of cloudiness, indicative of larger particle sizes and/or poor stability (Examples 1-3, respectively). In contrast, as described in Example 4, adding a mixture of lipid component, flavoring, surfactant, and active to a solution of buffer and water provided an emulsion with superior clarity and stability.

This is particularly surprising in that the relatively hydrophilic and water-soluble active ingredients utilized in the present disclosure would not have been expected to destabilize an emulsion. Further surprising is that the active ingredients (i.e., having a logP value in a range from about -0.5 to about 3) actually stabilize the emulsion when added as part of the SEDS component and reduce the quantity of emulsifying agent necessary to achieve a stable emulsion having a desirable particle size range.

Accordingly, in one aspect is provided a method of preparing an emulsion configured for oral use, the method comprising:
a) combining an emulsifying agent, a lipid component, a flavoring agent, and an active ingredient to form a self-emulsifying delivery system (SEDS) component;
b) providing a buffer solution comprising a buffering agent and a solvent; and
c) combining the SEDS component with the buffer solution to form the emulsion.
Each of said emulsifying agent, lipid component, flavoring agent, active ingredient, buffering agent, and solvent are as described herein above. In some embodiments, the method is performed in the exact order as recited.

In some embodiments, the flavoring agent is obtained as a solution in a suitable lipid, such as MCT oil. In some embodiments, the combining steps are each performed by stirring the respective components. In some embodiments, the emulsifying agent, lipid component, flavoring, and active ingredient are stirred together for a period of time at a speed in a range from about 100 to about 1000 rpm to form the SEDS component. In some embodiments, the SEDS and buffer solution are stirred together for a period of time at a speed in a range from about 100 to about 1000 rpm to form the oil-in-water emulsion.

Formation of emulsions via a SEDS component may be characterized as "low energy" emulsion formation, as generally, gentle mixing in the form of stirring is all that is necessary to provide an emulsion. In some embodiments, the emulsion is formed directly by combining the emulsion components without first forming a SEDS component. While formation of emulsions via SEDS component may be advantageous with respect to the low input energy required to form the emulsion and consequent low cost, this method may also be less advantageous in some embodiments. For example, as described herein, a relatively high water content may be required to provide emulsions having the desired properties and stability, generally require relatively larger concentrations of emulsifying agent, and may be more specific with respect to choice of emulsifying agent. In contrast, use of a high energy emulsification method may incur higher cost, but may also require less emulsifying agent, may be more permissive of emulsifying agent, may require less water, and may offer greater control over particle size. Accordingly, in another aspect is provided a method of preparing an emulsion configured for oral use, the method comprising combining an emulsifying agent, a lipid component, a flavoring agent, an active ingredient, a solvent, and optionally, further components such as salts, buffers, sweeteners, and the like to form the emulsion, wherein the combining comprises high-energy mixing. High-energy mixing utilizes mechanical devices (homogenizers) that are capable of generating intense disruptive forces that are capable of disrupting the lipid and solvent (e.g., oil and aqueous phases) into tiny oil droplets (see McClements and Rao, Critical Reviews in Food Science and Nutrition, 51, 285-330 (2011)). Such high-energy approaches include the use of high-pressure valve homogenizers, microfluidizers, and sonication methods. For example, emulsions as disclosed herein can be prepared by mechanical processes which employ shear force to break large emulsion droplets into smaller ones, such as high-pressure homogenization (HPH, including microfluidization), high-amplitude ultrasonic processing, and ultrasound-assisted emulsification. In some embodiments, the emulsion may be formed via the use of a high-pressure valve homogenizer, a microfluidizer, or an ultrasonic homogenizer (including ultrasonic jet homogenizers and ultrasonic probe homogenizers). In general, an emulsion of the present disclosure can be prepared by homogenizing a mixture of the various emulsion components (e.g., solvent, lipid component, active ingredient, emulsifying agent, and the like) with a homogenizer or mixer for a period of time. In some embodiments, the components are combined and homogenized with, for example, a probe sonicator (Sonics and Materials, USA), a high-pressure homogenizer (such as one made by Gauline or Avestine, or the like), or a microfluidizer. The number of passes through a high-pressure homogenizer/microfluidizer may vary, depending on the desired particle size for the emulsions. A variety of methods are known in the art for producing emulsions comprising nano-sized particles of particular size ranges, using for example, sonication or homogenization. One such method is described in U.S. Pat. No. 4,737,323, incorporated herein by reference.

In some embodiments, the emulsion, prepared via a SEDS or direct emulsification, is combined with one or more fillers, one or more sweeteners, one or more salts, or a combination thereof to form a composition. Accordingly, in some embodiments, the method further comprises contacting the emulsion with additional components to form the composition. Each of the fillers, sweeteners, and salts are as described herein above. In some embodiments, a filler, sweetener, and salt are combined to form a powder component (i.e., a premix), which is then combined with the emulsion. The components (filler, sweetener, salt, emulsion, and any other optional components) may be contacted, combined, or mixed together using any mixing technique or equipment known in the art as described above to form the composition. In some embodiments, a portion of the emulsion (e.g., up to about half) is added to the powder component followed by mixing, and then the remaining portion is added, followed by further mixing. In some embodiments, the resulting composition is in a free-flowing, particulate form.

In some embodiments, the composition is enclosed in a pouch as described herein above to form a pouched product. In some embodiments, the composition is introduced into the pouch material in the form of a particulate material (e.g., as a dry powder). Various manufacturing apparati and methods can be used to create a pouched product described herein. For example, US Publication No. 2012/0055493 to Novak, III et al., previously incorporated by reference in its entirety, relates to an apparatus and process for providing pouch material formed into a tube for use in the manufacture of oral products. Similar apparatuses that incorporate equipment for supplying a continuous supply of a pouch material (e.g., a pouch processing unit adapted to supply a pouch material to a continuous tube forming unit for forming a continuous tubular member from the pouch material) can be used to create a pouched product described herein. Representative equipment for forming such a continuous tube of pouch material is disclosed, for example, in U.S. Patent Application Publication No. US 2010/0101588 to Boldrini et al., which is incorporated herein by reference in its entirety. The apparatus further includes equipment for supplying pouched material to the continuous tubular member such that, when the continuous tubular member is subdivided and sealed into discrete pouch portions, each pouch portion includes a charge of a composition adapted for oral use. Representative equipment for supplying the filler material is disclosed, for example, in U.S. Patent Application Publication No. US 2010/0018539 to Brinkley, which is incorporated herein by reference in its entirety. In some instances, the apparatus may include a subdividing unit for subdividing the continuous tubular member into individual pouch portions and once subdivided into the individual pouch portions, may also include a sealing unit for sealing at least one of the ends of each pouch portion. In other instances, the continuous tubular member may be sealed into individual pouch portions with a sealing unit and then, once the individual pouch portions are sealed, the continuous tubular member may be subdivided into discrete individual pouch portions by a subdividing unit subdividing the continuous tubular member between the sealed ends of serially disposed pouch portions. Still in other instances, sealing (closing) of the individual pouch portions of the continuous tubular member may occur substantially concurrently with the subdivision thereof, using a closing and dividing unit. It is noted that in some embodiments of the present disclosure wherein a low melting point binder material is used, the temperature required for sealing the seams of the pouched product can be less than the temperature required in conventional processes associated with conventional binder materials.

The pouched product may be contacted with water and/or humectant to provide the desired moisture content of the final pouched product. The moisture content (e.g., water content) of the pouched product, prior to use by a consumer, may vary according to the desired properties. Typically, the pouched product, prior to insertion into the mouth of the user, is from about 5 to about 60% by weight of water (or moisture content), for example, from about 5 to about 15%, from about 15 to about 25%, from about 25 to about 40%, or from about 40 to about 60% water by weight, based on the total weight of the pouched product. In some embodiments, the pouched product has a water content from about 40 to about 60% by weight. In some embodiments, the desired amount of water and/or humectant is sprayed or dripped onto the pouch.

Many modifications and other embodiments will come to mind to one skilled in the art to which this disclosure pertains having the benefit of the teachings presented in the foregoing description. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

### EXAMPLES

Aspects of the present disclosure are more fully illustrated by the following examples, which are set forth to illustrate certain aspects of the present disclosure and are not to be construed as limiting thereof.

### Determination of pH

A sample (5.0±0.1 grams) of the relevant material (pouched product, SEDS component, or composition as disclosed herein) was placed in a specimen container. Deionized water (50±1 mL) was added, and the mixture stirred with a magnetic stir bar for 15 minutes. The pH was measured on a calibrated Orion Model 3 Combination Electrode and Meter.

### Particle size measurements

Particle size measurements for the droplets in the various emulsions were made on a Malvern Mastersizer 3000 (Malvern Instruments, UK). The droplets in the dispersed phase were assumed spherical with a refractive index of 1.46 and a density 0.95 (SEDS system properties measured on a standard refractometer). The average of the five measurements was recorded as the particle size.

In the case of pouched products, a pouch is placed in 3 mL of water at room temp (approximately 20°C) and allowed to stand without agitation for 60 minutes to extract pouch contents. The resulting emulsion in the form of an extract comprising components of the pouch composition is subjected to particle size analysis to indicate the presence and size ranges of the particles in the emulsion. Visual inspection is also used to indicate the presence of an emulsion.

### Example 1. Oil-in-water emulsion including nicotine

An oil-in-water emulsion was prepared. A lipid component, a flavoring agent, and an emulsifying agent, each in the quantities provided in **Table 2,** were combined and the mixture added to a mixture of nicotine, the buffer, sweetener, salt, and water, followed by stirring at 350 rpm to form the oil-in-water emulsion. The emulsion was observed to be opaque upon ocular examination.

**Table 2. Emulsion components and quantities**

| **Component** | **Percent by weight** |
|---|---|
| Lipid component (Medium chain triglyceride mixture) | 0.5-3 |
| Emulsifying agent (Tween^{®} 80) | 1-15 |
| Flavoring agent (Pineapple flavor package) | 0.1-3 |
| Nicotine | 1-15 |
| Water | 55-95 |
| Sodium chloride | 2.3 |
| Buffering agent (sodium bicarbonate) | 0.1-3 |

### Example 2. Oil-in-water emulsion including nicotine

An oil-in-water emulsion was prepared. A lipid component, a flavoring agent, and a surfactant, each in the quantities provided in **Table 2**, were combined and the mixture added to a solution of nicotine in water. To this mixture was added the buffer, sweetener, and salt, followed by stirring at 350 rpm to form the oil-in-water emulsion. The emulsion was observed to be very cloudy upon ocular examination.

### Example 3. Oil-in-water emulsion including nicotine

An oil-in-water emulsion was prepared. A lipid component, a flavoring agent, an emulsifying agent, and nicotine, each in the quantities provided in **Table 2,** were combined and the mixture added to a solution of buffer, sweetener, and salt in water, followed by stirring at 350 rpm to form the oil-in-water emulsion. The emulsion was observed to be cloudy upon ocular examination.

### Example 4. Oil-in-water emulsion including nicotine

An oil-in-water emulsion was prepared. A lipid component, a flavoring agent, an emulsifying agent, and nicotine, each in the quantities provided in **Table 2,** were combined by stirring at 350 rpm to form a pre-emulsion (self-emulsifying delivery system; "SEDS"). The SEDS was then added to a solution of buffer in water to form an emulsion. The sweetener and salt were then added to the emulsion followed by stirring at 350 rpm to finalize the oil-in-water emulsion solution. The emulsion was observed to be nearly clear upon ocular examination.

Particle sizes were obtained for the emulsions of Examples 1-4 as described above and are presented in **Table 3**. With reference to **Table 3**, the particle size distribution (PSD) analysis (D10, D50, D90) showed significant differences between the emulsions of Examples 1-4. Specifically, after 10 days, the emulsion of Example 4 comprised droplets having particles sizes in a range from about 23 (D10) to about 161 nm (D90), while the particle size range for Examples 1 to 3 was in a range from about 200 to about 492 nm (**Table 3**).

**Table 3. Particle size distributions**

| **Particle Size Category** | **Particle Size (nanometers)** | | | |
|---|---|---|---|---|
| | **Example 1** | **Example 2** | **Example 3** | **Example 4** |
| **D10** | 171 | 74 | 230 | 23 |
| **D50** | 307 | 209 | 349 | 61 |
| **D90** | 485 | 451 | 492 | 161 |

Based on a comparison of the emulsions prepared in Examples 1-4, it was determined that preparing a pre-mix of lipid, flavoring agent, surfactant, and nicotine first allowed nicotine to assist in emulsion formation. Without wishing to be bound by theory, it is believed that the nicotine is acting as an emulsifier or stabilizer when part of the pre-mix. In contrast, adding nicotine to an emulsion tended to break the emulsion. It was also determined that dissolving the salt in water before adding the pre-mix also prevented the salt from breaking the emulsion.

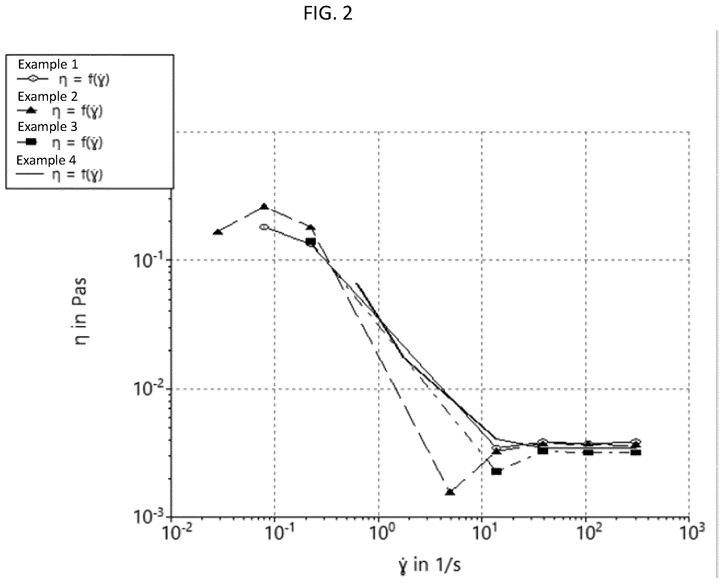

The viscosity of each of the emulsions of Examples 1-4 was measured on a HAAKE^{™} MARS^{™} iQ Air Rotational Rheometer (ThermoFisher Scientific, UK) with a lower plate and an upper cone (cone diameter: 35 mm; angle 2°) and a gap of 0.99 mm. All measurements were performed at 25 °C (1 replicate). Flow curves were obtained in the shear rate range of 0.01 to 300 s⁻¹ at 25 °C). A plot of viscosity versus shear rate is provided in **FIG. 2**, and viscosity values as a function of shear rate are provided as **Table 4**. With reference to **FIG. 2** and **Table 4**, all four samples showed a similar behaviour of a non-Newtonian fluid, demonstrating a decrease of viscosity with an increase of the shear rate (shear thinning).

**Table 4. Viscosity values (Pas) as a function of the Shear rate (s⁻¹)**

| **Example 1** | | **Example 2** | | **Example 3** | | **Example 4** | |
|---|---|---|---|---|---|---|---|
| **Shear rate (1/s)** | **Viscosity (Pas)** | **Shear rate (1/s)** | **Viscosity (Pas)** | **Shear rate (1/s)** | **Viscosity (Pas)** | **Shear rate (1/s)** | **Viscosity (Pas)** |
| 0.1 | 0.175 | 0.1 | 0.248 | 0.1 | 0.007 | 0.1 | -0.369 |
| 1 | -0.016 | 1 | -0.006 | 1 | -0.023 | 1 | 0.050 |
| 10 | 0.002 | 10 | 0.003 | 10 | 0.001 | 10 | 0.006 |
| 100 | 0.004 | 100 | 0.004 | 100 | 0.003 | 100 | 0.003 |
| 300 | 0.004 | 300 | 0.004 | 300 | 0.003 | 300 | 0.003 |

### Example 5. Eight-day Stability Study

Four oil-in-water emulsions were prepared (Examples 5A-5D). These corresponded to the preparative techniques of Examples 1-4, respectively, but each included additional water added after initial emulsion formation, and slightly different amounts of the various components as illustrated in **Table 5.**

For Example 5A, a lipid component, a flavoring agent, and an emulsifying agent, each in the quantities provided in **Table 5,** were combined and the mixture added to a mixture of nicotine, the buffer, sweetener, salt, and water, followed by stirring at 350 rpm to form the oil-in-water emulsion. Finally, additional water was added with stirring, providing a final water content in the emulsion of 47.3% by weight.

For Example 5B, a lipid component, a flavoring agent, and a surfactant, each in the quantities provided in **Table 5,** were combined and the mixture added to a solution of nicotine in water. To this mixture was added the buffer, sweetener, and salt, followed by stirring at 350 rpm to form the oil-in-water emulsion. Finally, additional water was added with stirring, providing a final water content in the emulsion of 47.3% by weight.

For Example 5C, a lipid component, a flavoring agent, an emulsifying agent, and nicotine, each in the quantities provided in **Table 5,** were combined and the mixture added to a solution of nicotine, buffer, sweetener, and salt in water, followed by stirring at 350 rpm to form the oil-in-water emulsion. Finally, additional water was added with stirring, providing a final water content in the emulsion of 47.3% by weight.

For Example 5D, a lipid component, a flavoring agent, an emulsifying agent, and nicotine, each in the quantities provided in **Table 5,** were combined by stirring at 350 rpm to form a SEDS. The SEDS was then added to a solution of buffer, sweetener, and salt in water, followed by stirring at 350 rpm to form the oil-in-water emulsion. Finally, additional water was added with stirring, providing a final water content in the emulsion of 47.3% by weight.

**Table 5. Emulsion components and quantities**

| **Component** | **Percent by weight** |
|---|---|
| Lipid component (Medium chain triglyceride mixture) | 0.2-5 |
| Emulsifying agent (Tween^{®} 80) | 1-15 |
| Flavoring agent (Pineapple flavor package) | 0.05-3 |
| Nicotine | 0.05-13 |
| Water (1) | 40.3 |
| Buffering agent (sodium bicarbonate) | 0.1-3 |
| Water (2) | 47.3 |
| Sodium Chloride | 3.9 |

### Example 6. Oil-in-water emulsions including nicotine; varying ratios of emulsifying agent to lipid component

A series of oil-in-water emulsions were prepared including varied ratios of emulsifying agent to lipid (Examples 6A-6C). Pineapple flavor was dissolved in MCT oil to form a flavorant solution containing 30% by weight of the flavorant in oil. This flavorant solution was added to Tween^{®} 80, nicotine, and water, each in the quantities provided in **Table 6.** The mixtures were combined by stirring at 350 rpm to form oil-in-water emulsions. The emulsion of Example 6A was opaque, and the emulsion of Example 6B was translucent but slightly cloudy. In contrast, the emulsion of Example 6C was completely clear.

**Table 6. Emulsion formulations**

| Example | Flavor plus MCT oil, total % by weight | Tween^{®} 80, % by weight | Nicotine, % by weight | Water, % by weight | Ratio of Tween^{®} 80 to Flavor plus MCT oil |
|---|---|---|---|---|---|
| 6A | 0.17 | 0.67 | 0.91 | 12.1 | 4 |
| 6B | 0.17 | 1.0 | 0.91 | 14.4 | 6 |
| 6C | 0.17 | 1.33 | 0.91 | 14.4 | 8 |

### Example 7. Oil-in-water emulsions including nicotine; varying amounts of water

A series of oil-in-water emulsions were prepared including varied amounts of water (Examples 7A-7C). Pineapple flavor was dissolved in MCT oil to form a flavorant solution containing 0.5% by weight of the flavorant in oil. This flavorant solution was added to Tween^{®} 80, nicotine, water, buffer, salt, and sweetener, each in the quantities provided in **Table 7.** The mixtures were combined by stirring at 350 rpm to form oil-in-water emulsions.

When the SEDS was added to bicarbonate dissolved in 20 parts water, along with sweetener and salt, a clear emulsion was obtained (7A). Utilizing 12 or 9 parts water (7B and 7C, respectively), produced cloudy and/or broken emulsions. The results of Examples 6 and 7 together demonstrate that dissolving sodium bicarbonate in water and subsequently adding it to the SEDS mitigates emulsion breakdown, and it is preferable to combine aqueous buffer with the SEDS prior to combining with further composition components such as fillers, sweeteners, and salts. Further, when sodium bicarbonate buffer was not dissolved in sufficient water, it disrupted emulsion stability, resulting in cloudy or broken emulsions.

**Table 6. Emulsion formulations**

| Sample | Pineapple flavor in MCT oil, % by weight | Tween^{®}80, % by weight | Nicotine, % by weight | Water, % by weight | Sodium bicarbonate, % by weight | Sucralose, % by weight | Sodium chloride, % by weight |
|---|---|---|---|---|---|---|---|
| 7A | 0.5 | 2.7 | 0.91 | 20 | 0.26 | 0.3 | 2.3 |
| 7B | 0.5 | 2.7 | 0.91 | 12 | 0.26 | 0.3 | 2.3 |
| 7C | 0.5 | 2.7 | 0.91 | 9 | 0.26 | 0.3 | 2.3 |

### Example 8. Pouched oral product

A pouched product according to a non-limiting embodiment of the disclosure was prepared using the formulation provided in **Table 8.** An oil-in-water emulsion was prepared. A lipid component, a flavoring agent, an emulsifying agent, and nicotine, each in the quantities provided in **Table 8,** were combined by stirring at 350 rpm to form a SEDS. The pineapple flavor package was utilized as a 0.5% solution in MCT oil. The SEDS was then added to a solution of buffer in water, followed by stirring at 350 rpm to form an oil-in-water emulsion.

A dry blend of filler, sweetener, and salt was prepared by mixing the three components as provided in **Table 8.** Approximately one half of the total volume of the emulsion was added to the dry blend, followed by mixing until homogenous (approximately 7 minutes). The remaining half of the emulsion was added to the mixture, followed by mixing until homogenous (approximately a further 7 minutes). The mixture was placed in pouches, which were sprayed with water to provide a total moisture content for the overall product of about 46%. Sensory evaluation of the pouches revealed a lack of noticeable flavor and aroma of pineapple.

**Table 8. Pouched product composition components and quantities**

| **Component** | **Percent by weight** |
|---|---|
| Emulsifying agent (Tween^{®} 80) | 4 |
| Flavor (pineapple) | 0.05 |
| Lipid component (MCT) | 0.95 |
| Nicotine (neat) | 0.5-1.5 |
| Buffer (sodium bicarbonate) | 0.2-0.3 |
| Water | 18-22 |
| Filler (microcrystalline cellulose) | 36-56 |
| Salt (sodium chloride) | 2-3 |
| Sweetener | 0.1-0.5 |
| water | 20-30 |

### Example 9. Pouched oral product

In view of the lack of perceived flavor in the pouch of Example 8, a pouched product according to a non-limiting embodiment of the disclosure was prepared using the formulation provided in **Table 9,** including a higher concentration of flavorant. An oil-in-water emulsion was prepared. A lipid component, a flavoring agent, an emulsifying agent, and nicotine, each in the quantities provided in **Table 9,** were combined by stirring at 350 rpm to form a SEDS. The pineapple flavor package was utilized as a 30% solution in MCT oil. The SEDS was then added to a solution of buffer in water, followed by stirring at 350 rpm to form an oil-in-water emulsion.

A dry blend of filler, sweetener, and salt was prepared by mixing the three components as provided in **Table 9.** Approximately one half of the total volume of the emulsion was added to the dry blend, followed by mixing until homogenous (approximately 7 minutes). The remaining half of the emulsion was added to the mixture, followed by mixing until homogenous (approximately a further 7 minutes). The mixture was placed in pouches, which were sprayed with water to provide a total moisture content for the overall product of about 46%. Sensory evaluation of the pouches revealed the desired flavor profile.

**Table 9. Pouched product composition components and quantities**

| **Component** | **Percent by weight** |
|---|---|
| Emulsifying agent (Tween^{®}80) | 2-3 |
| Flavor (pineapple) | 0.1-0.2 |
| Lipid component (MCT) | 0.3-0.4 |
| Nicotine (neat) | 0.5-1.5 |
| Buffer (sodium bicarbonate) | 0.2-0.3 |
| Water | 18-22 |
| Filler (microcrystalline cellulose) | 36-56 |
| Salt (sodium chloride) | 2-3 |
| Sweetener | 0.1-0.5 |
| water | 20-30 |

### Example 10. Pouched oral product-Heptanal

A pouched product according to non-limiting embodiments of the disclosure was prepared using the formulation provided in **Table 10.** An oil-in-water emulsion was prepared. A lipid component (MCT oil), heptanal as a volatile flavor component surrogate (35 micrograms per pouch), an emulsifying agent, and nicotine, each in the quantities provided in **Table 10,** were combined by stirring at 350 rpm to form a SEDS. The heptanal was utilized as a 1% by weight solution in MCT oil. The SEDS was then added to a solution of buffer in water, followed by stirring at 350 rpm to form an oil-in-water emulsion.

A dry blend of filler, sweetener, and salt was prepared by mixing the three components as provided in **Table 10.** Approximately one half of the total volume of the emulsion was added to the dry blend, followed by mixing until homogenous (approximately 7 minutes). The remaining half of the emulsion was added to the mixture, followed by mixing until homogenous (approximately a further 7 minutes). The mixture was placed in pouches, which were sprayed with water to provide a total moisture content for the overall product of about 46%.

**Table 10. Pouched product composition components and quantities**

| **Component** | **Percent by weight** |
|---|---|
| Emulsifying agent (Tween^{®} 80) | 2-3 |
| Heptanal (1% in MCT oil) | 0.4-0.6 |
| Nicotine (neat) | 0.5-1.5 |
| Buffer (sodium bicarbonate) | 0.2-0.3 |
| Water | 18-22 |
| Filler (microcrystalline cellulose) | 36-56 |
| Salt (sodium chloride) | 2-3 |
| Sweetener | 0.1-0.5 |
| water | 20-30 |

### Example 11. Pouched oral product-Heptanal (control)

A reference pouched product was prepared using the formulation provided in **Table 11.** The pouched product included MCT oil and heptanal (35 micrograms per pouch) as a volatile flavor component surrogate as in Example 10 but did not include an emulsifying agent or an emulsion. The mixture was placed in pouches, which were sprayed with water to provide a total moisture content for the overall product of about 46%.

**Table 11. Pouched product composition components and quantities**

| **Component** | **Percent by weight** |
|---|---|
| Heptanal (1% in MCT oil) | 0.4-0.6 |
| Nicotine (neat) | 0.5-1.5 |
| Buffer (sodium bicarbonate) | 0.2-0.3 |
| Water | 18-22 |
| Filler (microcrystalline cellulose) | 36-56 |
| Salt (sodium chloride) | 2-3 |
| Sweetener | 0.1-0.5 |
| water | 20-30 |

### Example 12. Analysis of heptanal content of Examples 10 and 11

The pouched products of Examples 10 and 11 were analyzed for heptanal content immediately after preparation (T0), and again at 15 days (T0.5), 30 days (T1), 60 days (T2), and 90 days (T3) after preparation. The amount of heptanal in each pouch for each timepoint is provided in **Table 12,** along with the ratio of concentration in the inventive pouch (Example 10) to the concentration in the reference pouch (Example 11). With reference to **Table 12,** the concentration of the volatile and lipophilic heptanal (logP of 2.44; vapor pressure of 3.85 mm Hg at 25°C) was generally 4 to 5 times greater in the inventive pouch (emulsion) than in the reference pouch (no emulsion).

**Table 12. Heptanal concentration over time**

| **Timepoint** | **Heptanal concentration (micrograms/pouch; mean, n=3)** | | |
|---|---|---|---|
| | **Example #11 (reference)** | **Example #10** | **Concentration Ratio, Example 10 to 11** |
| T0 | 0.7 | 3.5 | 5 |
| T0.5 | 0.5 | 2.0 | 4.1 |
| T1 | 0.5 | 1.8 | 3.6 |
| T2 | 0.5 | 2.0 | 3.8 |
| T3 | 0.3 | 1.1 | 4.0 |

### Example 13. Pouched oral product-pineapple flavoring agent

A pouched product according to non-limiting embodiments of the disclosure was prepared using the formulation provided in **Table 13.** An oil-in-water emulsion was prepared. A lipid component (MCT oil), pineapple flavor, an emulsifying agent, and nicotine, each in the quantities provided in **Table 13,** were combined by stirring at 350 rpm to form a SEDS. The SEDS was then added to a solution of buffer in water, followed by stirring at 350 rpm to form an oil-in-water emulsion.

A dry blend of filler, sweetener, and salt was prepared by mixing the three components as provided in **Table 13.** Approximately one half of the total volume of the emulsion was added to the dry blend, followed by mixing until homogenous (approximately 7 minutes). The remaining half of the emulsion was added to the mixture, followed by mixing until homogenous (approximately a further 7 minutes). The mixture was placed in pouches, which were sprayed with water to provide a total moisture content for the overall product of about 46%.

**Table 13. Pouched product composition components and quantities**

| **Component** | **Percent by weight** |
|---|---|
| Emulsifying agent (Tween^{®} 80) | 2-3 |
| Pineapple flavor (1% solution in MCT oil) | 0.4-0.6 |
| Nicotine (neat) | 0.5-1.5 |
| Buffer (sodium bicarbonate) | 0.2-0.3 |
| Water | 18-22 |
| Filler (microcrystalline cellulose) | 36-56 |
| Salt (sodium chloride) | 2-3 |
| Sweetener | 0.1-0.5 |
| water | 20-30 |

### Example 14. Pouched control oral product-Pineapple Flavor

A reference pouched product was prepared using the formulation provided in **Table 14.** The pouched product included MCT oil and pineapple flavor as in Example 13 but did not include an emulsifying agent or an emulsion. The mixture was placed in pouches, which were sprayed with water to provide a total moisture content for the overall product of about 46%.

**Table 14. Pouched product composition components and quantities**

| **Component** | **Percent by weight** |
|---|---|
| Pineapple flavor (1% solution in MCT oil) | 0.4-0.6 |
| Nicotine (neat) | 0.5-1.5 |
| Buffer (sodium bicarbonate) | 0.2-0.3 |
| Water | 18-22 |
| Filler (microcrystalline cellulose) | 36-56 |
| Salt (sodium chloride) | 2-3 |
| Sweetener | 0.1-0.5 |
| water | 20-30 |

### Example 15. Analysis of volatile ester content of Examples 13 and 14

The pouched products of Examples 13 and 14 were analyzed for the content of four major components contributing to the pineapple flavor (all low molecular weight esters). The analysis was performed at 40 days (T 1), and 70 days (T2) after preparation. The estimated amount present at the time of preparation (based on the flavoring agent amount added and the relative concentrations of each component therein), as well as the vapor pressure and logP of each of the four esters is provided in **Table 15.**

**Table 15. Pineapple flavor components and properties**

| **Ester#** | **Estimated amount added (micrograms/pouch)** | **Vapor pressure (mm Hg at 25°C)** | **logP** |
|---|---|---|---|
| **1** | 18 | 5.6 | 2.3 |
| **2** | 183 | 1.66 | 2.8 |
| **3** | 136 | 0.68 | 3.3 |
| **4** | 42 | 0.68 | 3.1 |

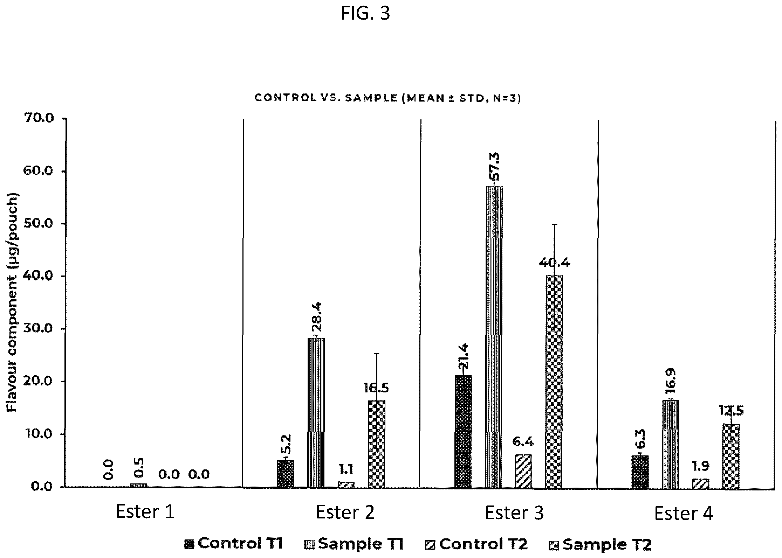

The amount of each of the four esters in each pouch for the 40 and 70-day timepoints is provided in **FIG. 3****.** With reference to **FIG. 3****,** the concentration of the four esters was higher in the inventive example than in the control example. Ester 1 (vapor pressure of 5.6 mm Hg) was not detectable in the control after 40 days age. The concentration of ester 2 was 5.5-fold higher in the inventive example than in the control at 40 days, and the concentration of esters 3 and 4 was 2.7-fold higher in the inventive example than in the control. These trends were also observed at 70 days. Overall, the stability/shelf-life of inventive pouch was slightly improved compared with the control as determined by retention of volatile esters over time (% losses 2-fold lower for the inventive Example 13 than the control Example 14). Without wishing to be bound by theory, it is believed that loss of esters over time was directly correlated with vapor pressure.

## Claims

1. A method of preparing an emulsion configured for oral use, the method comprising:
combining an emulsifying agent, a lipid component, a flavoring agent, and an active ingredient having a logP value in a range from about -0.5 to about 3.5, to form a self-emulsifying delivery system (SEDS) component;
providing a buffer solution comprising a buffering agent and a solvent; and
combining the SEDS component with the buffer solution to form the emulsion;
optionally, further comprising combining the emulsion with a powder component comprising one or more of a filler, a sweetener, and a salt to form a composition configured for oral use.

2. The method of claim 1, wherein the emulsifying agent has a hydrophilic-lipophilic balance (HLB) value in a range from about 3 to about 17, such as from about 3 to about 5, from about 8 to about 12, or from about 13 to about 17, and/or wherein the emulsifying agent comprises a polyethylene glycol monoester of a fatty acid; optionally, wherein the polyethylene glycol monoester of a fatty acid is a polyoxyethylene sorbitan monooleate, and preferably, wherein the polyoxyethylene sorbitan monooleate is polysorbate 20, polysorbate 40, polysorbate 60, polysorbate 80, or a combination thereof; and/or wherein the emulsifying agent comprises lecithin, gum acacia, modified gum acacia, a modified starch, or a combination thereof.

3. The method of claim 1 or claim 2, wherein the lipid component comprises medium chain triglycerides; optionally, wherein the lipid component comprises acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, okra seed oil, olive oil, orange oil, palm oil, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, soybean oil, sunflower oil, taramira oil, tea seed oil, thistle oil, tigernut oil, tobacco seed oil, tomato seed oil, walnut oil, watermelon seed oil, wheat germ oil, or a combination thereof; optionally, wherein the lipid component comprises castor oil, corn oil, coconut oil, cod liver oil, evening primrose oil, cottonseed oil, palm oil, rice bran oil, sesame oil, rapeseed oil, canola oil, linseed oil, olive oil, peanut oil, soybean oil, safflower oil, flaxseed oil, sunflower oil, olive oil, or a combination thereof.

4. The method of any one of claims 1-3, wherein one or more of the following applies:
the flavoring agent is lipophilic;
the flavoring agent comprises one or more volatile components, one or more components readily oxidizable upon exposure to air, or both; optionally, wherein the flavoring agent comprises heptanal;
the active ingredient is nicotine, a kavalactone, melatonin, or a combination thereof; optionally, wherein the active ingredient is nicotine;
the solvent comprises water, propylene glycol, glycerol, 1,2-propanediol, or a combination thereof; and/or
the buffering agent comprises a bicarbonate or carbonate alkali metal salt; preferably, wherein the buffering agent is sodium bicarbonate.

5. The method of any one of claims 1-4, wherein;
combining the emulsifying agent, the lipid component, the flavoring agent, and the active ingredient; and
combining the SEDS component with the buffer solution, each comprise stirring the respective components for a period of time at a speed in a range from about 100 to about 1000 rpm.

6. The method of any one of claims 1-5, wherein the emulsion comprises:
about 60% by weight or higher of the solvent, based on the total weight of the emulsion;
about 40% by weight or lower of the SEDS component, based on the total weight of the emulsion; and
wherein a ratio by weight of the emulsifying agent to the lipid component is in a range from about 3:1 to about 5:1.

7. The method of any one of claims 1-6, wherein the filler comprises a cellulose material, such as microcrystalline cellulose and/or wherein the composition comprises water in an amount from about 15 to about 25% by weight, based on the total weight of the composition.

8. The method of any one of claims 1-7, further comprising:
enclosing the composition in a pouch to form a pouched product; and
adding a quantity of water and/or humectant to the pouched product, wherein the quantity of water and/or humectant added is sufficient to provide an oven volatile content of the pouched product of about 5% to about 60% by weight, such as about 40% to about 60% by weight, based on the total weight of the pouched product.

9. An oral product comprising a composition, the composition comprising:
an emulsion comprising a solvent, a lipid component, an emulsifying agent having a hydrophilic-lipophilic balance (HLB) value in a range from about 3 to about 17, a lipophilic flavoring agent, and an active ingredient having a logP value in a range from about -0.5 to about 3.5;
one or more fillers; and
optionally, one or more sweeteners; one or more salts, and one or more buffering agents.

10. An oral product comprising a composition, the composition comprising:
an emulsion comprising a solvent, a lipid component, an emulsifying agent having a hydrophilic-lipophilic balance (HLB) value in a range from about 3 to about 17, a lipophilic flavoring agent, and an active ingredient, wherein a ratio by weight of the emulsifying agent to the lipid component is in a range from about 3:1 to about 5:1;
one or more fillers; and
optionally, one or more sweeteners; one or more salts, and one or more buffering agents.

11. The oral product of claim 9 or claim 10, comprising:
from about 30 to about 60% of the filler;
from about 40 to about 60% by weight of water;
from about 1 to about 5% by weight of emulsifying agent;
from about 0.25 to about 10% by weight of the lipid component;
from about 0.1 to about 10% by weight of active ingredient; and
from about 0.01 to about 2% by weight of the lipophilic flavoring agent, wherein each of the quantities by weight are based on the total weight of the oral product.

12. The oral product of any one of claims 9-11, wherein one of the following applies:
the lipid component comprises medium chain triglycerides;
the buffering agent is a carbonate or bicarbonate salt of an alkali metal; optionally, wherein the buffering agent is sodium bicarbonate;
the one or more fillers is a cellulose material; optionally, wherein the filler comprises microcrystalline cellulose;
the active ingredient comprises nicotine, a kavalactone, melatonin, resveratrol, or a combination thereof; optionally, wherein the active ingredient is nicotine; and/or a ratio by weight of the emulsifying agent to the lipid component is in a range from about 3:1 to about 5:1.

13. The oral product of any one of claims 9-12, wherein a combination of the flavoring agent and the lipid comprises the flavoring agent in an amount of about 20% by weight or more, based on a total combined weight of the lipid and the flavoring agent; optionally, wherein the combination of the flavoring agent and the lipid comprises the flavoring agent in an amount of about 25% or more or about 30% or more by weight, based on a total combined weight of the lipid and the flavoring agent.

14. The oral product of any one of claims 9-13, wherein one or more of the following applies:
the flavoring agent is lipophilic;
the flavoring agent comprises one or more volatile components;
the flavoring agent comprises one or more components readily oxidizable upon exposure to air;
the flavoring agent comprises one or more volatile components and one or more components readily oxidizable upon exposure to air;
the flavoring agent comprises heptanal;
the emulsifying agent comprises a polyethylene glycol monoester of a fatty acid;
the polyethylene glycol monoester of a fatty acid is a polyoxyethylene sorbitan monooleate;
the polyoxyethylene sorbitan monooleate is polysorbate 20, polysorbate 40, polysorbate 60, polysorbate 80, or a combination thereof; and/or
the emulsifying agent comprises lecithin, gum acacia, modified gum acacia, a modified starch, or a combination thereof.

15. The oral product of any one of claims 9-14, wherein the composition is enclosed in a water-permeable pouch to form a pouched product.
